# EUROPEAN PATENT SPECIFICATION

(11) **EP 4 205 774 B1**
(45) Date of publication and mention of the grant of the patent: **04.03.2026**
(21) Application number: 22217390.8
(22) Date of filing: 30.12.2022
(51) Int. Cl.: A61L 9/20, B60H 1/00, B60H 3/00, F24F 8/167, F24F 8/22, A61L 9/22, B01J 21/06, B01J 21/18, B01J 37/02

(54) **METHODS AND SYSTEMS FOR SANITIZING AIR CONDITIONED BY A CLIMATE CONTROL SYSTEM**
VERFAHREN UND SYSTEME ZUR DESINFEKTION VON DURCH EIN KLIMAREGELUNGSSYSTEM KONDITIONIERTER LUFT
PROCÉDÉS ET SYSTÈMES DE DÉSINFECTION DE L'AIR CONDITIONNÉ PAR UN SYSTÈME DE CLIMATISATION

(30) Priority: 31.12.2021 IN 202141062066
(43) Date of publication of application: 05.07.2023
(73) Proprietor: Thermo King LLC, Minneapolis, Minnesota 55420 (US)
(72) Inventor: BALAKRISHNA, Abhijith, 560061 Bangalore, Karnataka (IN); REDDY ALLURU, Sreedhar, 524406 Andhra Pradesh (IN); WANG, Gang, Holmen, Wisconsin, 54636 (US); POLYZOIS, Christos Alkiviadis, Bloomington, Minnesota, 55438 (US)
(74) Representative: Haseltine Lake Kempner LLP

(56) References cited:
- CN-B- 109 210 635
- CN-U- 205 939 546
- CN-U- 211 146 657
- US-A1- 2009 176 053

## Description

### FIELD

This disclosure relates generally to a climate control system. More specifically, this disclosure relates to methods and systems for sanitizing air conditioned by a climate control system.

### BACKGROUND

A climate control system can include, for example, a heating, ventilation and air conditioning (HVAC) system and/or a transport refrigeration system (TRS). An HVAC system is generally used to control a climate within a passenger space of a vehicle (e.g., a passenger bus, a cabin of a tractor, etc.) and/or residential HVAC applications and commercial HVAC applications. A TRS is generally used to control an environmental condition (e.g., temperature, humidity, air quality, and the like) within a cargo space of a transport unit (e.g., a truck, a container (such as a container on a flat car, an intermodal container, etc.), a box car, a semi-tractor, a bus, or other similar transport unit). The TRS can maintain environmental condition(s) of the cargo space to maintain cargo (e.g., produce, frozen foods, pharmaceuticals, etc.).

Society's vulnerabilities to a rapidly spreading certain microbes, such as, viruses and pathogens were exposed during the COVID-19 pandemic. Operators who use HVAC and TRS systems (commercial, industrial and residential) have different challenges to address the pathogen spread, for example, more complicated building and space design, an increased populous and densities of people, the increased movement of people worldwide and the general increasing interconnectedness of people worldwide, as well as the technologies associated with accommodating these complications and increases. The operators turn to policies, procedures, and operations and use technology to recommend appropriate remediation methods. However, as people are in close proximity, within an enclosed space in the transit vehicle or residential or commercial building, or have multiple interactions with others, these conditions can facilitate the spread of communicable diseases and pathogens such as COVID-19. Thus, legitimate hygienic solutions that foster confidence in various forms of transport, housing, and commerce are a benefit to public health and safety.

CN205939546 U discloses a fresh air regenerating device using a multistage photocatalyst. CN211146657 U discloses a box type warmer with an air purification function comprising a box body, an air inlet formed in the rear side of the box body, an air outlet formed in the front side of the box body, an air duct assembly arranged at the position, connected with the air outlet, in the box body, a heating device arranged in the air duct assembly, and a draught fan arranged at the tail end of the airduct assembly. CN109210635 B discloses air purification equipment capable of conducting targeted and efficient air purification according to the air quality.

### SUMMARY

This disclosure is directed to methods and systems for sanitizing air conditioned by a climate control system.

The embodiments described herein can provide an effective air sanitization solution for use in climate control applications. Accordingly, volatile organic compounds and microbes including, for example, viruses, pathogens, and bacteria, can be effectively reduced in climate control applications.

It will be appreciated that the embodiments described herein can be applied to transport climate control applications as well as both residential HVAC applications and commercial HVAC applications.

In one embodiment, an air sanitizer unit is provided according to claim 1. It may be that the substrate structure defining the channels is configured such that microbes in the airflow are captured on the at least one photocatalytic coating at low airflow rates. The low airflow rates may be airflow rates less than 20 air changes per hour.

In another embodiment, a climate control system is provided according to claim 9.

A method for sanitizing air is also provided according to claim 10.

### BRIEF DESCRIPTION OF THE DRAWINGS

References are made to the accompanying drawings that form a part of this disclosure, and which illustrate embodiments in which the systems and methods described in this Specification can be practiced.
FIG. 1A illustrates a perspective view of a mass-transit vehicle including a transport climate control system, according to one embodiment.
FIG. 1B illustrates a perspective view of a vehicle with an APU, according to an embodiment.
FIG. 1C illustrates a perspective view of a climate controlled transport unit attached to a tractor, according to one embodiment.
FIG. 1D illustrates a perspective view of a building including a climate control system, according to one embodiment.
FIG. 2 illustrates block diagram schematic of a mass-transit vehicle that includes an air sanitization system and a transport climate control system, according to one embodiment.
FIG. 3 is a perspective view, partially cutaway, illustrating an air handling unit of an HVACR system having a centrifugal fan, according to an embodiment.
FIG. 4 is a schematic view of an air sanitizer unit, not according to the invention.
FIG. 5 is a schematic view of a substrate for an air sanitizer unit, according to an embodiment.
FIG. 6 is a schematic view of a substrate for an air sanitizer unit, according to another embodiment.
FIG. 7 is a schematic view of a substrate for an air sanitizer unit, not according to the invention.
FIGS. 8A and 8B are schematic views of a substrate for an air sanitizer unit, not according to the invention.
FIG. 9A is a schematic view of a substrate for an air sanitizer unit, not according to the invention.
FIG. 9B is a schematic view of a substrate for an air sanitizer unit, not according to the invention.
FIGS. 10A, 10B, 10C are schematic views of a substrate for an air sanitizer unit, according to another embodiment.
FIGS. 11A and 11B are schematic views of a substrate for an air sanitizer unit, according to another embodiment.
FIG. 12 is a schematic view of a substrate for an air sanitizer unit, not according to the invention.
FIG. 13 is a schematic view of a substrate for an air sanitizer unit, not according to the invention.
FIG. 14 is a schematic view of a substrate for an air sanitizer unit, not according to the invention.
FIG. 15 is a schematic view of a substrate for an air sanitizer unit, not according to the invention.
FIG. 16 is a schematic view of an air sanitizer unit, not according to the invention.
FIG. 17 is a block flow diagram of a method for sanitizing air, according to an embodiment.

Like reference numbers represent like parts throughout.

### DETAILED DESCRIPTION

This disclosure is directed to methods and systems for sanitizing air conditioned by a climate control system.

It will be appreciated that the embodiments described herein can be applied to transport climate control applications as well as both residential HVAC applications and commercial HVAC applications.

Particular embodiments of the present disclosure are described herein with reference to the accompanying drawings; however, it is to be understood that the disclosed embodiments are merely examples of the disclosure, which can be embodied in various forms. Well-known functions or constructions are not described in detail to avoid obscuring the present disclosure in unnecessary detail. Therefore, specific structural and functional details disclosed herein are not to be interpreted as limiting, but merely as a basis for the claims and as a representative basis for teaching one skilled in the art to variously employ the present disclosure in virtually any appropriately detailed structure.

As used in this disclosure, volatile organic compounds (VOCs) include organic chemicals that have a high vapor pressure, e.g., in a gaseous state, at room temperature. VOCs can be emitted as gases from a wide array of solids or liquids. Examples can include, for example, paints and lacquers, paint strippers, cleaning supplies, pesticides, building materials and furnishings, office equipment such as copiers and printers, correction fluids and carbonless copy paper, graphics and craft materials including glues and adhesives, permanent markers, and photographic solutions. Microbes are microorganisms that include bacteria, viruses, fungi, and protozoa, in which the microbes that cause diseases are referred to as pathogens. A virion includes the entire virus particle, including an outer protein shell and an inner core of nucleic acid.

A climate control system is generally used to control one or more environmental conditions such as, but not limited to, temperature, humidity, air quality, or combinations thereof, of an internal space of, for example, a residential building, a commercial building, a transport unit, etc. Examples of transport units include, but are not limited to a truck, a container (such as a container on a flat car, an intermodal container, a marine container, a rail container, etc.), a box car, a semi-tractor, a mass-transit vehicle (such as a passenger bus, a passenger train, etc.), or other similar transport unit. A climate controlled transport unit can be used to transport perishable items such as pharmaceuticals, produce, frozen foods, and meat products and/or can be used to provide climate comfort for passengers in a passenger space of a mass-transit vehicle. The climate control system can also be used in both residential HVAC applications and commercial HVAC applications to control one or more environmental conditions such as, but not limited to, temperature, humidity, air quality, or combinations thereof, of a residential or commercial building to provide climate comfort for occupants in the residential and/or commercial building. The climate control system may include a vapor-compressor type climate controlled system, a thermal accumulator type system, or any other suitable climate controlled system that can use a working fluid (e.g., refrigerant, etc.), cold plate technology, or the like.

A climate control system can include a climate control unit (CCU) to control one or more environmental conditions (e.g., temperature, humidity, air quality, etc.) of a climate controlled space. The CCU can include, without limitation, a climate control circuit (including, for example, a compressor configured to compress a working fluid (e.g., refrigerant), a condenser, an expansion valve, and an evaporator), and one or more fans or blowers to control the heat exchange between the air within the climate controlled space and the ambient air outside of the climate controlled space.

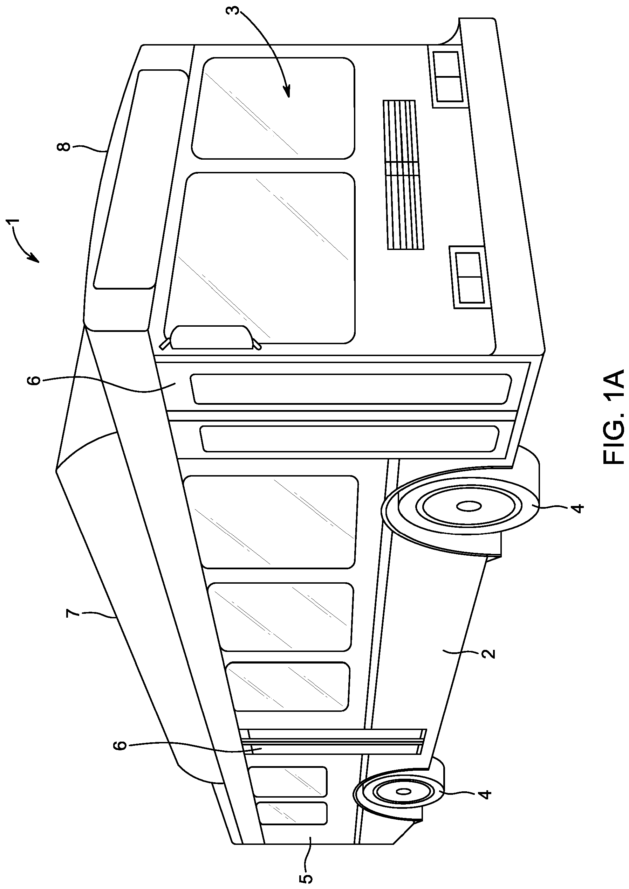

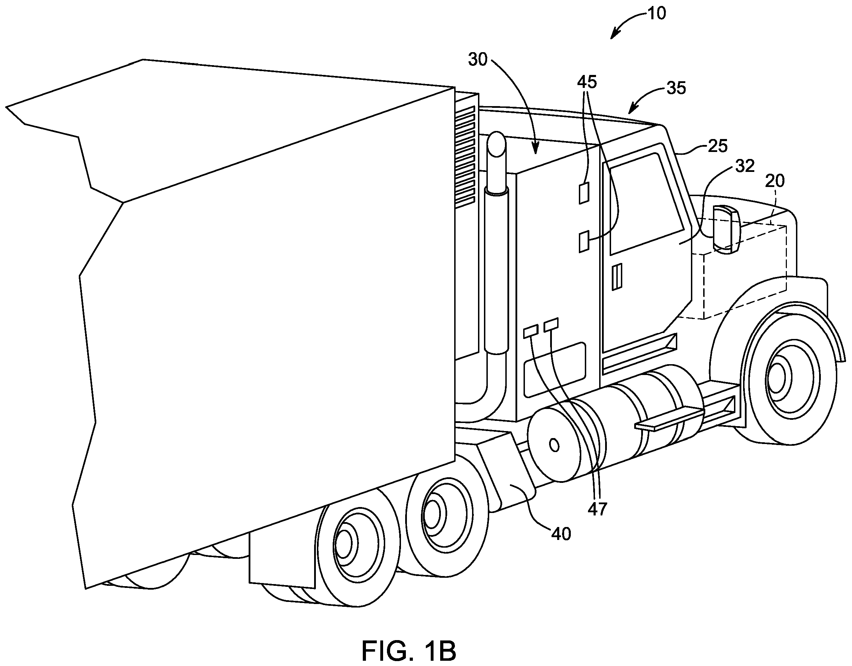

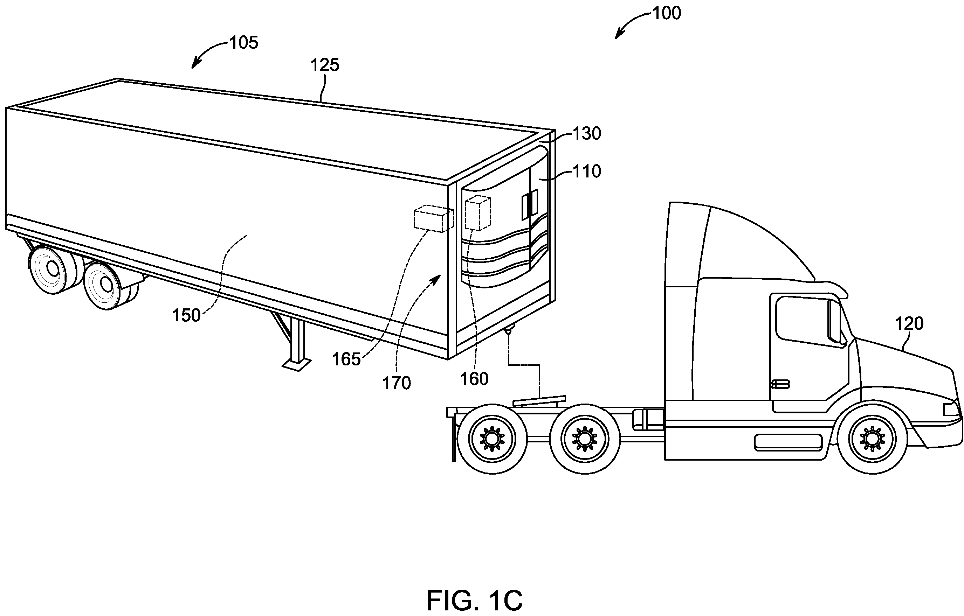

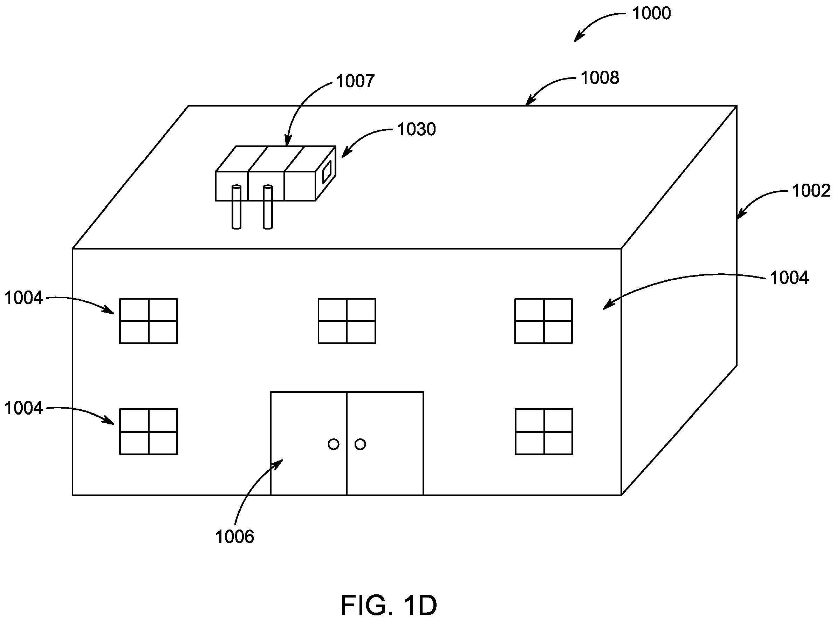

FIGS. 1A, 1B, and 1C show various transport climate control systems. It will be appreciated that the embodiments described herein are not limited to the examples provided below, but can apply to any type of transport unit (e.g., a truck, a container (such as a container on a flat car, an intermodal container, a marine container, etc.), a box car, a semi-tractor, a passenger bus, or other similar transport unit), etc., as well as being used for residential HVAC applications and commercial HVAC applications. With respect to a HVAC application, FIG. 1D shows a climate control system for a building employing the embodiments described herein.

FIG. 1A is a perspective view of a mass-transit vehicle 1 including a climate control system, according to one embodiment. In the embodiment illustrated in FIG. 1A, the vehicle 1 is a mass-transit bus that can carry passenger(s) (not shown) to one or more destinations. In other embodiments, the vehicle 1 can be a school bus, railway vehicle, subway car, or other commercial vehicle that carries passengers. Hereinafter, the term "mass-transit vehicle" shall be used to represent all such vehicles, and should not be construed to limit the scope of the application solely to mass-transit buses.

FIG. 1A shows that the vehicle 1 includes a frame 2, a passenger compartment 3 supported by the frame 2, wheels 4, and a compartment 5. The frame 2 includes doors 6 that are positioned on a side of the vehicle 1. As shown in FIG. 1A, a first door 6 is located adjacent to a forward end of the vehicle 1, and a second door 6 is positioned on the frame 2 toward a rearward end of the vehicle 1. Each door 6 is movable between an open position and a closed position to selectively allow access to the passenger compartment 3.

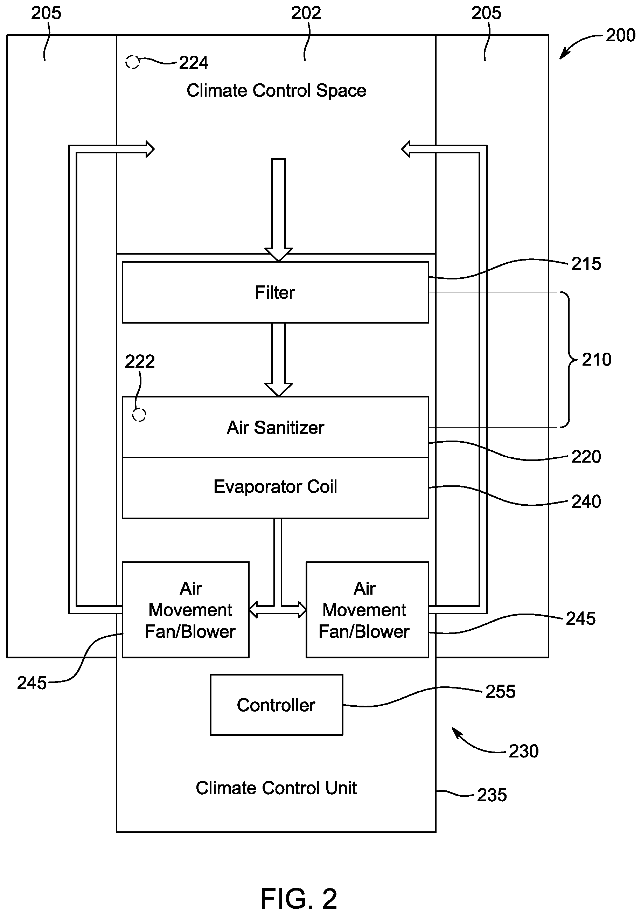

The vehicle 1 also includes a climate control unit 7 attached to the frame 2 on a roof 8 of the vehicle 1. The climate control unit 7 is part of a transport climate control system (not shown) that is configured to provide climate control within the passenger compartment 3. In some embodiments, the climate control unit 7 can include a climate control circuit (not shown) with one or more fans/blowers to provide climate conditioned air within the passenger compartment 3. The climate control unit 7 can be combined with an air sanitization system (see FIG. 2) that is configured to purify air within the passenger compartment 3. While the climate control unit 7 is shown as a rooftop mount onto the roof 8, it will be appreciated that in other embodiments the climate control unit 7 can be located at other sides of the vehicle 1 (e.g., mounted to a rear end of the vehicle 1).

The compartment 5 is located adjacent the rear end of the vehicle 1, can include a power system (not shown) that is coupled to the frame 2 to drive the wheels 4. In some embodiments, the compartment 5 can be located in other locations on the vehicle 1 (e.g., adjacent the forward end, etc.).

FIG. 1B illustrates a vehicle 10 according to one embodiment. The vehicle 10 is a semi-tractor that is used to transport cargo stored in a cargo compartment (e.g., a container, a trailer, etc.) to one or more destinations. Hereinafter, the term "vehicle" shall be used to represent all such tractors and trucks, and shall not be construed to limit the invention's application solely to a tractor in a tractor-trailer combination. In some embodiments, the vehicle 10 can be, for example, a straight truck, van, etc.

The vehicle 10 includes a primary power source 20, a cabin 25 defining a sleeping portion 30 and a driving portion 35, an auxiliary power unit (APU) 40, and a plurality of vehicle accessory components 45 (e.g., electronic communication devices, cabin lights, a primary and/or secondary HVAC system, primary and/or secondary HVAC fan(s), sunshade(s) for a window/windshield of the vehicle 10, cabin accessories, etc.). The cabin 25 can be accessible via a driver side door (not shown) and a passenger side door 32. The cabin 25 can include a primary HVAC system (not shown) that can be configured to provide conditioned air within driving portion 35 and potentially the entire cabin 25, and a secondary HVAC system (not shown) for providing conditioned air within the sleeping portion 30 of the cabin 25. In some embodiments, the secondary HVAC system can be combined with an air sanitization system (see FIG. 2) that is configured to purify air within the sleeping portion 30 of the cabin 25. The cabin 25 can also include a plurality of cabin accessories (not shown). Examples of cabin accessories can include, for example, a refrigerator, a television, a video game console, a microwave, device charging station(s), a continuous positive airway pressure (CPAP) machine, a coffee maker, a secondary HVAC system for providing conditioned air to the sleeping portion 30.

The primary power source 20 can provide sufficient power to operate (e.g., drive) the vehicle 10 and any of the plurality of vehicle accessory components 45 and cabin accessory components 47. The primary power source 20 can also provide power to the primary HVAC system and the secondary HVAC system. In some embodiments, the primary power source can be a prime mover such as, for example, a combustion engine (e.g., a diesel engine, etc.).

The APU 40 can include a plurality of energy storage elements each of which is coupled to one of a plurality of converters. The converters can provide electric power (e.g., AC or DC power) generated by the APU 40 to one or more vehicle accessory components, cabin accessory components, a primary HVAC system, and a secondary HVAC system. A secondary HVAC system can provide conditioned air to a sleeping portion of a vehicle cabin (e.g., the sleeping portion 30 of the cabin 25 shown in FIG. 1B). The energy storage elements can be, for example, battery packs, fuel cells, etc. In some embodiments, the APU 40 can be turned on or off by an occupant (e.g., driver or passenger) of the vehicle. For example, the occupant can turn on the APU 40 to provide power stored in the energy storage elements when a primary power source of the vehicle is turned off. It will be appreciated that the embodiments described herein can also be used with a prime mover powered APU. In some embodiments, APU 40 can include a vehicle electrical system.

FIG. 1C illustrates one embodiment of a climate controlled transport unit 105 attached to a tractor 110. The climate controlled transport unit 105 includes a climate control system 100 for a transport unit 125. The tractor 120 is attached to and is configured to tow the transport unit 125. The transport unit 125 shown in FIG. 1C is a trailer. It will be appreciated that the embodiments described herein are not limited to tractor and trailer units, but can apply to any type of transport unit (e.g., a container on a flat car, an intermodal container, etc.), a truck, a box car, or other similar transport unit. The transport unit 125 can include one or more doors (not shown) that are movable between an open position and a closed position to selectively allow access to a climate controlled space 150.

The climate control system 100 includes a climate control unit (CCU) 110 that provides environmental control (e.g. temperature, humidity, air quality, etc.) within the climate controlled space 150 of the transport unit 125. The climate control system 100 also includes a climate controller 170 and one or more sensors (not shown) that are configured to measure one or more parameters of the climate control system 100 and communicate parameter data to a climate controller 170.

The CCU 110 is disposed on a front wall 130 of the transport unit 125. In other embodiments, it will be appreciated that the CCU 110 can be disposed, for example, on a rooftop or another wall of the transport unit 125. The CCU 110 includes a climate control circuit (not shown) for conditioning air to be provided within the climate controlled space 150. The CCU 110 can also include a power system to power components of the climate control system 100 (e.g., a compressor, one or more fans and blowers, one or more sensors, one or more solenoid valves, etc.). The CCU 110 can be combined with an air sanitization system (see FIG. 2) that is configured to purify air within the climate controlled space 150.

The programmable climate controller 170 may comprise a single integrated control unit 160 or that may comprise a distributed network of climate controller elements 160, 165. The number of distributed control elements in a given network can depend upon the particular application of the principles described herein. The climate controller 170 is configured to control operation of the climate control system 100.

FIG. 1D is a perspective view of a building 1000 including a climate control system, according to one embodiment. In the embodiment illustrated in FIG. 1D, the building 1000 is a commercial office building having multiple occupants that come and go throughout the day. In other embodiments, the building 1000 can be a residential building, hospital, nursing home, manufacturing building, grocery store, retail store, church or other religious building, or the like. and should not be construed to limit the scope of the application solely to the described buildings.

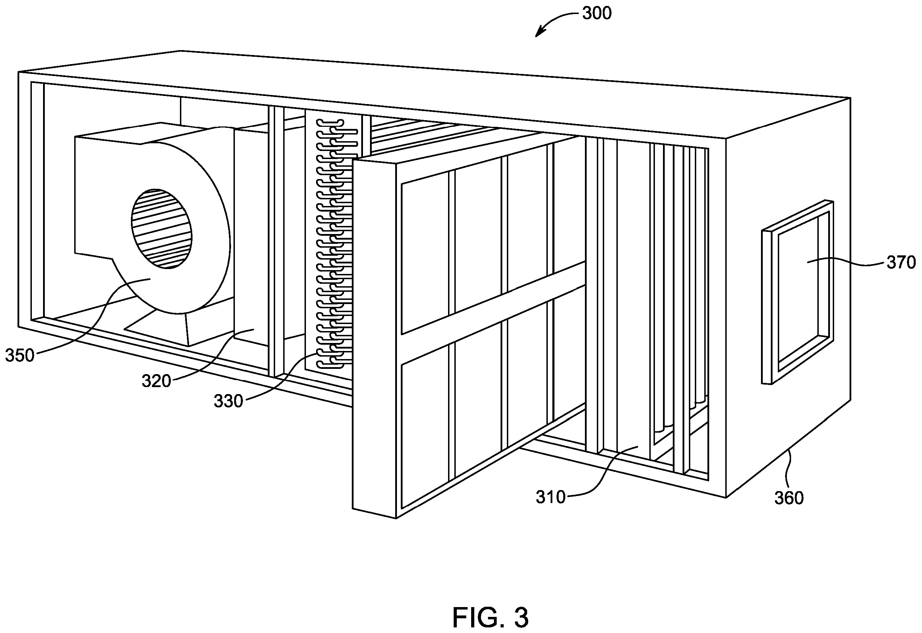

FIG. 1D shows that the building 1000 includes a frame 1002 and multiple offices 1004. The frame 1002 includes a door 1006 that allows egress and ingress into the building 1000. The building 1000 also includes a climate control unit 1007 attached to the frame 1002 on a roof 1008 of the building 1000. The climate control unit 1007 is part of a climate control system 1030 that is configured to provide climate control to the multiple offices 1004 and the building 1000. In some embodiments, the climate control unit 1007 can include a climate control circuit (not shown) with one or more fans/blowers to provide climate conditioned air within the multiple offices 1004 and the building 1000. The climate control unit 1007 can be combined with an air sanitization system (see FIGS. 2 and 3) that is configured to purify air within the multiple offices 1004 and the building 1000. While the climate control unit 1007 is shown as a rooftop unit on the roof 1008, it will be appreciated that in other embodiments the climate control unit 1007 can be located at other positions of the building 1000 (e.g., mounted below the building 1000 or adjacent thereto on the ground).

FIG. 2 illustrates block diagram schematic of a mass-transit vehicle 200 that includes an air sanitization system 210 and a transport climate control system 230, according to one embodiment. The mass-transit vehicle 200 can be, for example, the mass-transit vehicle 1 shown in FIG. 1A. The mass-transit vehicle 200 includes a climate controlled space 202 for transporting one or more passengers travelling in the mass-transit vehicle 200. The mass-transit vehicle 200 can be, for example, a mass-transit bus (e.g., a school bus, a city bus, etc.), a railway vehicle, a subway car, or other mass-transit vehicle that carries passengers.

It will be appreciated that the embodiments described herein with respect to FIG. 2 are not limited to a mass-transit vehicle and can be used with other transport units including, for example, the vehicle 10 shown in FIG. 1B, the climate controlled transport unit 105 shown in FIG. 1C, etc. It will also be appreciated that the embodiments described herein with respect to FIG. 2 can also be applied in non-transport applications such as the building 1000 shown in FIG. 1D (e.g., residential HVAC applications, commercial HVAC applications, etc.)

The mass-transit vehicle 200 also includes two air delivery ducts 205. The climate controlled space 202 can be a passenger compartment for passengers travelling in the mass-transit vehicle 200. The climate controlled space 202 can optionally include one or more contamination sensors 224 that are configured to monitor air quality (e.g., contamination) within the climate controlled space 202. The one or more contamination sensors 224 can include one or more indoor air quality (IAQ) sensors and/or one or more contaminant sensors. In some embodiments, the one or more IAQ sensors can measure, for example, CO₂, total volatile organic compounds, particulate matter, temperature, humidity, etc. within the climate controlled space 202. In some embodiments, the one or more contaminant sensors can measure and identify specific species of contaminants in the air.

The air delivery ducts 205 are in airflow communication with both the climate controlled space 202 and a climate control unit 235 of the transport climate control system 230. The air delivery ducts 205 are configured to distribute climate controlled air generated by the climate control unit 235 into the climate controlled space 202. Each of the air delivery ducts 205 can include multiple openings (not shown) to distribute the climate controlled air substantially evenly within the climate controlled space 202. While the mass-transit vehicle 200 includes two air delivery ducts 205, it will be appreciated that the number of air delivery ducts 205 can vary as required by the mass-transit vehicle 200. For example, in another embodiment, the mass-transit vehicle 200 can include only a single air delivery duct 205. In some embodiments, the mass-transit vehicle 200 can also include one or more fresh air dampers (not shown) that can provide fresh air (e.g., ambient air outside of the mass-transit vehicle 200) downstream of an evaporator coil 240 of the climate control unit 235. It will be appreciated that the mass-transit vehicle 200 can also include one or more doors and/or windows (not shown) that can also introduce fresh air into the climate controlled space 202.

The air sanitization system 210 includes a filter 215 and an air sanitizer unit 220. The filter 215 can include a filter media that is configured to trap contaminants (e.g., dust particles, etc.) from an airflow passing through the filter 215. In some embodiments, the filter media can be a fabric filter media. In some embodiments, the filter 215 can have a multi-layer construction. In some embodiments, an anti-microbial coating can be applied to the filter 215. In some embodiments, the filter 215 can be a replaceable filter. It will be appreciated that the size of particles that can be trapped by the filter 215 can vary, for example, based on a minimum efficiency reporting values (MERV) rating. In some embodiments, the filter 215 can be a MERV 7 filter. In some embodiments, the filter 215 can be an electrostatic filter.

The air sanitizer unit 220 is configured to purify and/or sanitize the airflow that has first passed through the filter 215 and is passing through a climate control unit 235 of the transport climate control system 230. In particular, the air sanitizer unit 220 can oxidize and/or decompose organic pollutants, such as VOCs and microbes of airborne viruses and bacteria, in the airflow passing there through. The air sanitizer unit 220 includes a substrate having channels for allowing the airflow to pass there through, in which a substrate structure of the substrate defines the channels that can include a photocatalytic catalyst coating, such as, a photocatalytic oxidation (PCO) air purifier, that can create hydroxyl radicals, H₂O₂, ions, super-oxides ions, or the like that can oxidize and/or decompose any VOCs and/or microbes, in the airflow passing there through. In particular, the PCO air purifier includes a light-activated catalyst configured to react with organic pollutants to oxidize and/or decompose them into a non-toxic substance. The substrate structure that defines the channels is provided such that the microbes are further captured on the photocatalytic coating, even at low airflow rates, so that the surface area of the photocatalytic coating that is exposed to the light from a light source is further able to oxidize and/or decompose the pollutants.

In some embodiments, the PCO air purifier can be a graphene based PCO air purifier (GPCO) that uses a graphene enhanced titanium oxide catalyst to generate local hydroxyl radicals and super-oxide ions on the surface of the catalyst that can oxidize and/or decompose the organic pollutants when they come in contact with the air sanitizer 220. In some embodiments, the GPCO air purifier can use an ultraviolet (UV) light to excite and activate the catalyst (e.g., a graphene enhanced titanium oxide catalyst) to begin the chemical reaction. The use of graphene can increase the amount of surface area that the titanium oxide is applied to and thus create more surface area for UV light to shine, thereby increasing the amount of hydroxyl radicals and/or super-oxide ions generated. In some embodiments, the UV light can be generated using one or more light emitting diodes (LEDs), one or more mercury lamps, etc. In some embodiments, the UV light can generate ~390 nm light (e.g., when using one or more LEDs, the UV light generated can spike at around 395 nm). An advantage of using LEDs is that they can be safer than other types of light sources such as, for example, mercury light bulbs which can become dangerous should the mercury leak out of or otherwise be removed from the light bulb. Another advantage is that the GPCO air purifiers do not capture or store any of the compounds that contact the graphene enhanced titanium oxide catalyst. Thus, the graphene enhanced titanium oxide catalyst does not become loaded or consumed and does not require periodic replacement or maintenance.

In some embodiments, the PCO (or GPCO) air purifier of the air sanitizer unit 220 can include multiple LED panels. In some embodiments, each of the LED panels can include multiple LED circuits. For example, in one embodiment, each LED panel can include four LED circuits. In some embodiments, each of the multiple LED panels of the air sanitizer unit 220 can be independently controlled (e.g., turned ON or OFF) such that not all of the LED panels are ON at the same time. In some embodiments, the air sanitizer unit 220 can be a low voltage device that require, for example, ~24 VDC or less in order to operate.

In some embodiments, the air sanitizer unit 220 can include, for example, an ultraviolet germicidal irradiation (UVGI) air purifier, a bi-polar ionizer, a dry hydrogen peroxide generator, an ozone generator, etc.

It will be appreciated that in embodiments where the air sanitizer unit 220 emits, for example, hydroxyls, certain species of contaminants (e.g., halogens such as chlorine, fluorine, etc.) can become more toxic or dangerous when reacting with the hydroxyls (e.g., when oxidized). Accordingly, there can be embodiments whereby the air sanitizer unit 220 can be turned OFF when a level of certain species of contaminants are monitored by one or more contaminant sensors (of the one or more contamination sensors 224).

The air sanitization unit 220 can optionally include one or more operational sensors 222 that can monitor operation of the air sanitizer unit 220 to ensure that the air sanitizer unit 220 is operating properly. In some embodiments, the one or more operational sensors 222 are configured to monitor one or more operational parameters of the air sanitizer unit 220. In some embodiments, the one or more operational sensors 222 can include one or more current sensors configured to monitor a current drawn by each of the multiple LED panels and/or each of the multiple LED circuits. In some embodiments, the one or more operational sensors 222 can include one or more power sensors configured to monitor a power drawn by each of the multiple LED panels and/or each of the multiple LED circuits. In some embodiments, the one or more operational sensors 222 can include one or more UV light sensors configured to monitor an intensity of the UV light. In some embodiments, the one or more operational sensors 222 can include one or more lumen sensors configured to monitor a luminance of each of the multiple LED panels and/or each of the multiple LED circuits. In some embodiments, the one or more operational sensors 222 can be replaced or used in conjunction with, for example, the one or more contamination sensors 224.

In some embodiments, transport climate control system 230 and the air sanitization system 210 are configured such that a majority if not an entire portion of the air flow passing over the evaporator coil 240 must also pass through the air sanitizer unit 220. In some embodiments, this can be achieved by mounting the air sanitizer unit 220 directly to the evaporator coil 240 such that the air sanitizer unit 220 covers the entire evaporator coil 240. In these embodiments, a majority if not the entire airflow can either pass through the air sanitizer unit 220 first before passing over the evaporator coil 240 or pass over the evaporator coil 240 first before passing through the air sanitizer unit 220. In some embodiments, the air sanitizer unit 220 can be located at other locations of the passenger vehicle 200 such as anywhere in the air delivery ducts 205. In these embodiments, the air sanitizer unit 220 can also be configured such that a majority if not the entire portion of the air flow directed to the climate controlled space 202 passes through the air sanitizer unit 220.

It will be appreciated that the mass-transit vehicle 200 can have high airflow rates within the climate controlled space 202. Applicant has found that hydroxyls, H2O2, ions, or the like that are emitted from typical PCO air purifiers may not survive long enough to travel from, for example, the evaporator coil 240, to the air movement fans/blowers 245, through the air delivery ducts 205 and into the climate controlled space 202 because of the length of travel and the high airflow rate. By providing an embodiment in which the air sanitizer unit 220 covers the entire evaporator coil 240, the air sanitization system 210 can require that all airflow passing over the evaporator coil 240 is first directed through the air sanitizer unit 220 to ensure that all conditioned air entering the climate controlled space is purified as it passes through the air sanitizer unit 220. Accordingly, any hydroxyls, H₂O₂, ions, or the like that are emitted by the air sanitizer unit 220 are not required or intended to travel to and enter the climate controlled space 202 in order to attack any volatile and/or organic compounds in the air provided in the climate controlled space 202. This can prevent any harmful and/or any organic compounds or ozone from being emitted by the air sanitization system 210 into the climate controlled space 202.

The transport climate control system 230 includes the climate control unit 235, a controller 255, and a plurality of climate control sensors (not shown). In some embodiments, the transport climate control system 230 can also include a human machine interface (HMI) configured to allow a user to manually communicate with and/or provide instructions to the transport climate control system 230, a telematics unit configured to wirelessly connect the transport climate control system 230 to user(s) and/or remote server(s) that are remote from the mass-transit vehicle 200. The climate control unit 235 includes an evaporator coil 240 and a plurality of air movement fans/blowers 245. The climate control unit 235 can also include a climate control circuit (not shown) that includes, for example, a compressor configured to compress a working fluid (e.g., a refrigerant), a condenser coil, the evaporator coil 240, and an expansion valve. The climate control circuit can include other components that are known in the art for conditioning air to be directed to a climate controlled space (e.g., one or more valves, a receiver tank, an economizer, working fluid lines, etc.). The climate control unit 235 can also include one or more condenser fans configured to direct air in a heat exchange relationship with the condenser coil out of the climate control unit 235 into the ambient outside of the mass-transit vehicle 200. The climate control sensors can be provided throughout the mass-transit vehicle 200 and monitor one or more climate control parameters. The climate control sensors, for example, can include: a return air temperature sensor that monitors the temperature of air returned from the climate controlled space 202 back to the climate control unit 235; a supply air temperature sensor that monitors the temperature of air supplied by the CCU 235 into the climate controlled space 122; a humidity sensor that monitors the humidity within the climate controlled space 202; an ambient temperature sensor that monitors the temperature outside of the mass-transit vehicle 200; a compressor suction pressure sensor that monitors a pressure at or near a suction port of the compressor; a compressor discharge pressure sensor that monitors a pressure at or near a discharge port of the compressor; etc.

The air movement fans/blowers 245 can be, for example, evaporator fans or blowers that are configured to direct conditioned air that has undergone a heat exchange while passing over the evaporator coil 240 into the air delivery ducts 205. It will be appreciated that the number of air movement fans/blowers 245 can vary based on the needs of the transport climate control system 230. For example, in some embodiments, the climate control unit 235 can include only a single air movement fans/blowers 245. In some embodiments, the air movement fans/blowers 245 can operate at multiple non-zero speeds (e.g., a low speed, a medium speed, and a high speed). In some embodiments, the low speed can be in a range of ~30-63% of a maximum speed of the air movement fans/blowers 245. In some embodiments, the medium speed can be in a range of ~55-75% of a maximum speed of the air movement fans/blowers 245. In some embodiments, the high speed can be in a range of ~65-88% of a maximum speed of the air movement fans/blowers 245. In some embodiments, the air movement fans/blowers 245 can be variable speed air movement fans/blowers that operate along a gradient range of non-zero speeds. In some embodiments, the air movement fans/blowers 245 can be low voltage devices that require, for example, ~24 VDC or less in order to operate.

The controller 255 is configured to control operation of the transport climate control system 230 and can also control operation of the air sanitization system 210. In particular, the controller 255 is in electrical communication with the air movement fans/blowers 245, the compressor, the one or more condenser fans, the one or more valves, etc. Accordingly, the controller 255 can control operation of the transport climate control system by controlling operation of the air movement fans/blowers 245, the compressor, the one or more condenser fans, valves, etc. That is, the controller 255 can control whether the air movement fans/blowers 245, the compressor, the one or more condenser fans, one or more valves, etc. are ON or OFF. The controller 255 can also control a speed of the air movement fans/blowers 245, the compressor, the one or more condenser fans, etc. Also, the controller 255 can control the size of the opening of the one or more valves. The controller 255 is configured to receive climate control parameter data from the one or more climate control sensors. It will be appreciated that the controller 255 can use the climate control parameter data to control operation of the transport climate control system 230 and the air sanitization system 210.

In some embodiments, the controller 255 can also control operation of the air sanitizer unit 220. In particular, the controller 255 is in electrical communication with the air sanitizer unit 220. Accordingly, the controller 255 can turn the air sanitizer unit 220 ON or OFF as required and control the amount of power directed to the air sanitizer unit 220. In some embodiments, the controller 255 can selectively turn ON and OFF or limit the power provided to individual LED panels of the air sanitizer unit 220 while the air sanitizer unit 220 is in operation. For example, in some embodiments, the controller 255 can selectively turn OFF or limit power directed to a certain number of the LED panels while the air sanitizer unit 220 is in operation in order to reduce the purification capacity of the air sanitizer unit 220. The controller 255 can selectively turn OFF or reduce power to a certain number of the LED panels while the air sanitizer unit 220 is operating in order to reduce energy consumption of the air sanitizer unit 220 during, for example, periods when the mass-transit vehicle 200 may be lightly loaded, the risk of contamination in the climate controlled space is low, etc. Also, the controller 255 can rotate which of the multiple LED panels are ON and OFF or have reduced power to achieve uniform aging of the air sanitizer unit 220. In some embodiments, the controller 255 is configured to receive operational parameter data from the one or more operational sensors 222.

In some embodiments, the controller 255 can control the transport climate control system 230 and the air sanitization system 210 in a variety of operation modes. These operation modes, for example, can include: a continuous cooling mode whereby the compressor is continuously operating to provide cooling to the climate controlled space 202; a start-stop cooling mode whereby the compressor is periodically turned ON and OFF while providing cooling to the climate controlled space 202; a heating mode to provide heating to the climate controlled space 202; a defrost mode to defrost the evaporator coil 240; an air sanitization mode to purify air in the climate controlled space 202 by operating the air movement fans/blowers 245 at a high speed; etc.

In some embodiments, the controller 255 can be in communication with the mass-transit vehicle 200, a HMI of the transport climate control system 230, a telematics unit of the transport climate control system 230, etc. In some embodiments, the controller 255 can receive a communication signal from the mass-transit vehicle 200 when the mass-transit vehicle has been turned ON. For example, the controller can receive a signal from the mass-transit vehicle 200 that an ignition switch of the mass-transit vehicle 200 has been turned ON.

FIG. 2 shows arrows to illustrate how an airflow is directed through the mass-transit vehicle 200, according to one embodiment. In particular, air in the climate controlled space travels into climate control unit 235 and passes through the filter 215. The airflow passing through the filter 215 is then directed through the air sanitizer unit 220 to be purified and then over the evaporator coil 240 to be conditioned. The air movement fans/blowers 245 then directs the purified and conditioned airflow past the evaporator coil and into the air delivery ducts 205. The air delivery ducts 205 then return purified and conditioned air back into the climate controlled space 202.

FIG. 3 is a perspective view, partially cutaway, illustrating an air handling unit (air handler) 300 of an HVACR system having a centrifugal fan 350, according to an embodiment. The HVACR system can be used for HVAC systems for residential applications or for commercial applications, for example, the building 1000 shown in FIG. 1D.

The unit 300 includes an enclosure or housing 360. In one embodiment, the enclosure 360 can be a generally rectangular cabinet having a first end wall defining an air inlet opening 370 (to allow air to flow into an internal space of the enclosure 360) and a second end wall defining an air outlet opening (not shown, to allow air to flow out of the enclosure 360 via an air outlet (that overlaps with the air outlet opening) of the centrifugal fan 350. In FIG. 3, a side wall of the enclosure 360 is cutaway and the internal space of the enclosure 360 is shown.

The unit 300 also includes a primary filter 310 and a secondary filter. In one embodiment, the primary filter 310 and the secondary filter can be one filter. It will be appreciated that the primary filter 310 and/or the secondary filter can be a porous device configured to remove impurities or solid particles from air flow passed through the device.

The unit 300 further includes a component (e.g., a coil) 330. In one embodiment, the component 330 can be an air conditioning evaporator coil disposed in the flow path of air passing from the air inlet opening 370 to the air outlet opening of the enclosure 360 (which is also the air outlet of the fan 350). It will be appreciated that the component 330 can be different types in that the working fluid can be e.g., refrigerant, water, or the like. For example, when the working fluid is refrigerant, the component 330 can be an evaporator coil for cooling, and/or can be a condenser coil for heating. For example, when the working fluid is water, the component 330 can be tube(s) for chilled water to go through for cooling, and can be tube(s) for hot water to go through for heating.

The unit 300 also includes an air sanitizer unit 320. The air sanitizer unit 320 is configured to purify and/or sanitize the airflow that has first passed through the filter 310 and is passing over the component 330. In particular, the air sanitizer unit 320 can oxidize and/or decompose organic pollutants, such as, VOCs and microbes of airborne viruses and bacteria, in the airflow passing there through. The air sanitizer unit 320 includes a substrate having channels for allowing the airflow to pass there through, in which a substrate structure of the substrate defines the channels that can include a photocatalytic catalyst coating, such as, a photocatalytic oxidation (PCO) air purifier, that can create hydroxyl radicals, H₂O₂, ions, super-oxides ions, or the like that can oxidize and/or decompose any VOCs and/or microbes in the airflow passing there through. The substrate structure that defines the channels is provided such that the microbes are further captured on the photocatalytic coating, even at low airflow rates, so that the surface area of the photocatalytic coating that is exposed to the light from a light source is further able to oxidize and/or decompose the pollutants.

In some embodiments, the air sanitizer unit 320 can be the graphene based PCO air purifier (GPCO) that uses a graphene enhanced titanium oxide catalyst to generate local hydroxyl radicals and super-oxide ions on the surface of the catalyst that can oxidize and/or decompose the organic pollutants when they come in contact with the air sanitizer 320. In some embodiments, the GPCO air purifier can use an ultraviolet (UV) light to excite and activate the catalyst (e.g., a graphene enhanced titanium oxide catalyst) to begin the chemical reaction. In some embodiments, the UV light can be generated using one or more light emitting diodes (LEDs), one or more mercury lamps, etc. In some embodiments, the UV light can generate ~390 nm light (e.g., when using one or more LEDs, the UV light generated can spike at around 395 nm).

In some embodiments, the GPCO air purifier of the air sanitizer unit 320 can include multiple LED panels. In some embodiments, each of the LED panels can include multiple LED circuits. For example, in one embodiment, each LED panel can include four LED circuits. In some embodiments, each of the multiple LED panels of the air sanitizer unit 220 can be independently controlled (e.g., turned ON or OFF) such that not all of the LED panels are ON at the same time. In some embodiments, the air sanitizer unit 320 can be a low voltage device that require, for example, ~24 VDC or less in order to operate.

In some embodiments, HVACR system and the air sanitization unit 320 are configured such that a majority if not an entire portion of the air flow passing over the component 330 must also pass through the air sanitizer unit 320. In some embodiments, this can be achieved by mounting the air sanitizer unit 320 directly adjacent the component 330 such that the air sanitizer unit 320 covers the entire component 330. In these embodiments, a majority if not the entire airflow can either pass through the air sanitizer unit 320 after passing over the component 330 or pass over the component 330 first before passing through the air sanitizer unit 320.

Also the unit 300 includes a fan (or blower) 350. In one embodiment, the fan 350 can be a centrifugal fan having electric drive motor (not shown) to drive the fan 350 (e.g., to drive a shaft of the fan 350, to rotate the impeller of the fan 350). It will be appreciated that a centrifugal fan is a mechanical device for moving air or other gases toward the outlet of the fan in a direction at an angle (e.g., perpendicular) to the incoming air from the inlet of the fan. A centrifugal fan often contains a ducted housing to direct outgoing air in a specific direction or across a heat sink. The centrifugal fan can increase the speed and volume of an air stream with rotating impellers.

In some embodiments, the air sanitizer unit 320 can be located at other locations of the air handling unit, in which a majority if not all of the airflow flows through the air sanitizer unit 320. For example, the fan (or blower) 350 can be provided near the inlet 370 and passes the airflow through the air sanitizer unit 320.

The air sanitizer units 220, 320 as discussed above, exhibit good performance against volatile organic compounds (VOCs) across a wide range of airflow rates, e.g., between 1 and 100 air changes per hour (ACH). Applicant found, however, that while at high airflow rates, e.g., 50 ACH or higher, the PCO and/or GPCO air purifier exhibited good performance against microbes, for example, Bacteriophage MS2 virus, Staphylococcus aureus bacterium, at low airflow rates, the antimicrobial performance of the PCO and/or GPCO air purifier decreased. Since the low airflow rates are typically found in residential and commercial buildings and the airflow rate of transport units, for example, the mass-transit vehicle 200, can be decreased to the low airflow rates, e.g., by decreasing the fan (or blower) speed, the antimicrobial performance of the air sanitizer units 220, 320 need to be improved so that the air sanitizer units 220, 320 are able to purify and/or sanitize the airflow over a wide range of airflow rates, and most preferably, at low airflow rates of less than 40 ACH, preferably less than 30 ACH, and most preferably between 5 and 20 ACH.

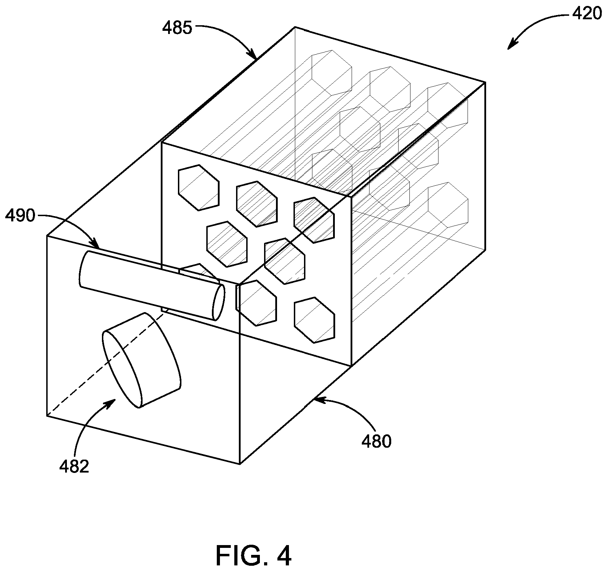

FIG. 4 shows a schematic representation of an unmodified air sanitizer unit 420 which can be used in any of the systems as discussed above with respect to air sanitizer units 220, 320. The air sanitizer unit 420 includes a housing 480, a substrate 485 provided in an interior space of the housing 480, and a light source 490 disposed within the interior space of the housing 480. The housing 480 can include an inlet 482 for receiving the airflow from the climate controlled space and/or a filter and an outlet (not shown) to discharge the purified and/or sanitized airflow to be conditioned and/or back into the climate controlled space. It is appreciated that the inlet 482 can be a hole or aperture provided on the front face of the air sanitizer unit 420 or can be the entirety of the front face for receiving the airflow.

The substrate 485 includes at least one photocatalytic coating on at least one surface that extends along a flow path of the airflow through the air sanitizer unit 420. For example, without intending to be limiting in scope, the substrate 485 can have a honeycomb structure, in which the honeycomb structure includes a plurality of cells that define channels for allowing airflow there through that extend along the flow path of the airflow through the air sanitizer unit 420. The plurality of cells can include hexagonal tubes that are covered with photocatalytic catalyst. The light source 490 is disposed within the interior space of the housing upstream of the substrate structure in a way such that light produced by the light source activates the at least one photocatalytic coating to purify and/or sanitize the airflow. The light source 490 can use an ultraviolet (UV) light to excite and activate the catalyst to begin the chemical reaction.

As discussed above, while prior designs of the air sanitizer unit and the unmodified air sanitizer unit 420, exhibit good performance against volatile organic compounds (VOCs) across a wide range of airflow rates, the air sanitizer unit 420 was not found to have good antimicrobial performance against certain organic compounds at low airflow rates, e.g., less than 40 ACH, preferably less than 30 ACH, and most preferably between 5 and 20 ACH.

Specifically, without wishing to be bound by theory, it is understood that the reduction of organic contaminants, such as, VOCs and microbes, by the air sanitizer unit can occur through the combination of three phenomena: steering of the contaminants to the catalyst; retention of the contaminants by the catalyst; and oxidation of the contaminants. The steering of the organic contaminant molecules to the catalyst surface can occur through the Brownian motion of these gas molecules. The retention of the organic contaminant molecules by the catalyst happens through adsorption, which is significantly enhanced by the graphene. The oxidation of the organic contaminants occurs by the reactive oxygen species (primarily hydroxyl radicals OH and superoxide anions O₂⁻) produced by the photocatalytic process, in which the hydroxyls tend to stay close to the surface of the catalyst.

When the airflow rate is decreased, there are fewer passes through the air sanitizer unit. The purification and/or sanitization of the VOC in the airflow is primarily unaffected by the decrease in airflow rate, at least because, for VOCs, each pass may have increased efficiency, since the VOC gas molecules spend more time inside the tubes, the VOC gas molecules are steered to the surface of the catalyst through Brownian motion, where they will be adsorbed. However, the purification and/or sanitization of the microbes in the airflow can be affected by low airflow rates, at least because of the size of the microbe in comparison to the size of other organic molecules, such as VOCs. For example, the size of a VOC gas molecule, for example, a formaldehyde molecule, is about 0.25 nm, while the size of a microbe, for example, a pathogen, such as, the MS2 virus (which is a small virus), is 27 nm. In other words, the size of the microbe that is the pathogen is 100 times larger than the VOC gas molecule, and has a volume ratio of 1,000,000:1. Further, when one considers that virions that form the entire virus particles are rarely found individually and that they are typically found in aerosolized particles of size 1 µm or larger, the volume of aerosolized particles carrying the microbe may be 64 billion (or more) times larger than that of VOC molecules. Therefore, the air sanitizer unit does not have the benefit of Brownian motion to steer the aerosolized particles having the microbe to the surface of the catalyst for reduction and/or oxidation of the microbe, but instead, the air sanitizer unit relies solely on the number of passes through the air sanitizer unit for reduction of the microbe. Thus, the antimicrobial efficiency of the air sanitizer unit drops as the airflow rates decreases.

The following designs of the air sanitizer unit according to embodiments of the disclosure are designed so that the air sanitizer unit is able to capture and reduce and/or oxidize microbes even at low airflow rates by having a substrate structure that defines channels for allowing airflow there through and include the photocatalytic coating, in which the substrate structure that defines the channels is configured such that microbes in the airflow are captured on the photocatalytic coating even at low airflow rates. In order to more clearly discuss the features of the various embodiments having the improved antimicrobial efficiency, only the structure of the substrate used in the air sanitizer unit, for example, the air sanitizer unit 420, is discussed below. It is appreciated that the various embodiments of the substrate can be used in any of the air sanitizer units 220, 320, 420 and any of the systems as discussed above with respect to air sanitizer units 220, 320, in which any feature not discussed with respect to the air sanitizer unit can thus include the same or similar features as the air sanitizer units 220, 320, 420.

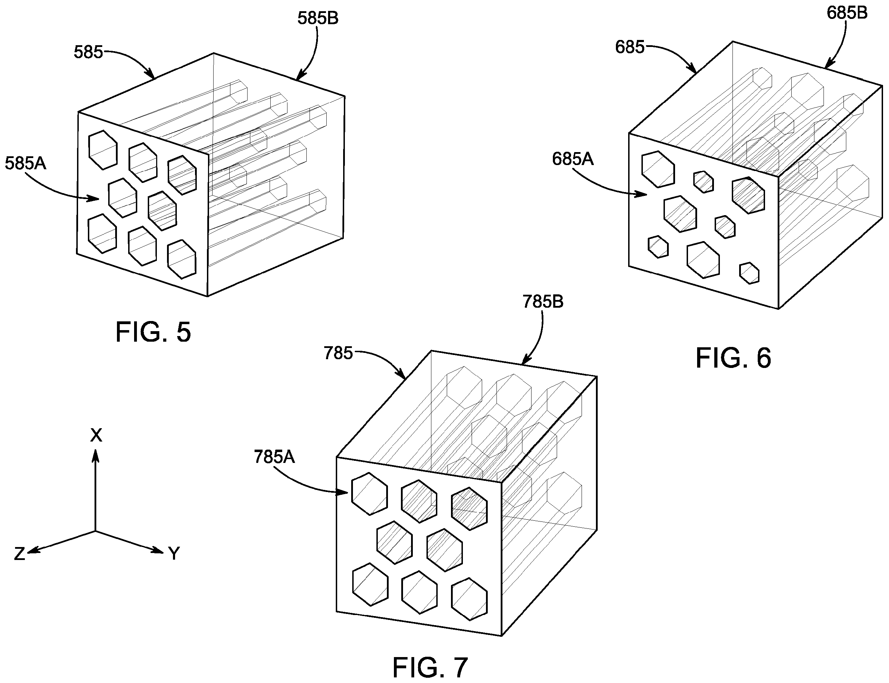

FIG. 5 shows a schematic representation of a substrate according to an embodiment. The substrate 585 has a honeycomb structure that includes a plurality of cells that define channels that extend along the flow path of the airflow. The plurality of cells defining the channels can include hexagonal tubes that are covered with the photocatalytic catalyst. The honeycomb structure can be a metal substrate, and can include metals such as aluminum, copper, steel, stainless, steel, alloys thereof, or combinations thereof or a ceramic or plastic substrate, or similar materials that are usable for photocatalytic oxidation substrates. The at least one photocatalytic coating can include a photocatalytic catalyst that can create hydroxyl radicals, H₂O₂, ions, super-oxides ions, or the like that can oxidize and/or decompose any VOCs and microbes. In some embodiments, the at least one photocatalytic coating is a graphene enhanced titanium oxide catalyst to generate local hydroxyl radicals and super-oxide ions on the surface of the catalyst that can oxidize and/or decompose VOCs and microbes when they come in contact with the substrate 585.

As seen in FIG. 5, the plurality of cells has a structure in which the hexagonal tubes have an inlet end 585A and an outlet end 585B. The inlet end 585A of the hexagonal tubes has a cell diameter that is larger than the cell diameter at the outlet end 585B. For example, the cells at the outlet end 585B can have a cell diameter that is 1/4, 1/3, 1/2, 3/4, or the like the size of the cell diameter at the inlet end 585A. That is, the hexagonal tubes taper along the channels from the inlet end 585A to the outlet end 585B. In view of such structure, it is appreciated that the substrate structure defining the channels, at least in part due to the narrowing/tapering of the tubes which restricts airflow, has a high deposition rate and retention rate of aerosolized particles, e.g., capturing of the particles, that include the microbes on the photocatalytic coating catalyst, even at low airflow rate, e.g., less than 40 ACH, preferably less than 30 ACH, and most preferably between 5 and 20 ACH. By having such features, the surface area of the photocatalytic coating catalyst having the aerosolized particles that include the microbes is increased, so that the photocatalytic coating catalyst can further oxidize and/or decompose the microbes, e.g., due to the increased surface area, exposure to the light source is increased to further activate the photocatalytic coating catalyst and neutralize the virions.

It is appreciated that while the plurality of cells has been discussed relative to hexagonal shaped tubes, it is understood that the tubes can have various geometries, for example, circular, triangular, rectangular, trapezoidal, octagonal, or the like.

FIG. 6 shows a schematic representation of a substrate according to another embodiment. The substrate 685 has a honeycomb structure that includes a plurality of cells that define channels that extend along the flow path of the airflow. The plurality of cells defining the channels can include hexagonal tubes that are covered with the photocatalytic catalyst. The honeycomb structure can be a metal substrate, and can include metals such as aluminum, copper, steel, stainless, steel, alloys thereof, or combinations thereof or a ceramic or plastic substrate, or similar materials that are usable for photocatalytic oxidation substrates. The at least one photocatalytic coating can include a photocatalytic catalyst that can create hydroxyl radicals, H₂O₂, ions, super-oxides ions, or the like that can oxidize and/or decompose any VOCs and microbes. In some embodiments, the at least one photocatalytic coating is a graphene enhanced titanium oxide catalyst to generate local hydroxyl radicals and super-oxide ions on the surface of the catalyst that can oxidize and/or decompose VOCs and microbes when they come in contact with the substrate 685.

As seen in FIG. 6, the plurality of cells has a structure in which the hexagonal tubes have an inlet end 685A and an outlet end 685B. A first plurality of the cells at the inlet end 685A of the hexagonal tubes has cells having a cell diameter that is larger than the cell diameter of the same cells at the outlet end 685B. For example, the cells at the outlet end 685B can have a cell diameter that is 1/4, 1/3, 1/2, 3/4, or the like the size of diameter of the same cells at the inlet end 685A. A second plurality of the cells at the inlet end 685A has a cell diameter that is smaller than the diameter of the same cell at the outlet end 685B. For example, cells at the inlet end 685A can have a cell diameter that is 1/4, 1/3, 1/2, 3/4, or the like the size of the diameter of the cells at the outlet end 685B. That is, the first plurality of cells has hexagonal tubes that taper so that the channels narrow from the inlet end 685A to the outlet end 685B, while the second plurality of cells has hexagonal tubes that taper so that the channels widen from the inlet end 685A to the outlet end 685B. In view of such structure, it is appreciated that the substrate structure defining the channels, at least in part due to the tapering of the tubes, has a high deposition rate and retention rate of aerosolized particles that include the microbes on the photocatalytic coating catalyst, even at low airflow rate, e.g., less than 40 ACH, preferably less than 30 ACH, and most preferably between 5 and 20 ACH. By having such features, the surface area of the photocatalytic coating catalyst having the aerosolized particles that include the microbes is increased, so that the photocatalytic coating catalyst can further oxidize and/or decompose the microbes, e.g., due to the increased surface area, exposure to the light source is increased to further activate the photocatalytic coating catalyst and neutralize the virions.

FIG. 7 shows a schematic representation of a substrate according to another embodiment. The substrate 785 has a honeycomb structure that includes a plurality of cells that define channels that extend along the flow path of the airflow. The plurality of cells defining the channels can include hexagonal tubes that are covered with the photocatalytic catalyst. The honeycomb structure can be a metal substrate, and can include metals such as aluminum, copper, steel, stainless, steel, alloys thereof, or combinations thereof or a ceramic or plastic substrate, or similar materials that are usable for photocatalytic oxidation substrates. The at least one photocatalytic coating can include a photocatalytic catalyst that can create hydroxyl radicals, H₂O₂, ions, super-oxides ions, or the like that can oxidize and/or decompose any VOCs and microbes. In some embodiments, the at least one photocatalytic coating is a graphene enhanced titanium oxide catalyst to generate local hydroxyl radicals and super-oxide ions on the surface of the catalyst that can oxidize and/or decompose VOCs and microbes when they come in contact with the substrate 785.

As seen in FIG. 7, the plurality of cells has a structure in which the hexagonal tubes have an inlet end 785A and an outlet end 785B. The hexagonal tubes are angled, e.g., offset at an angle, with respect to the cells at the inlet end 785A and the outlet end 785B. It is appreciated that the hexagonal tubes can be angled between 30 degrees and 60 degrees in the X or Y directions. In view of such structure, it is appreciated that the substrate structure defining the channels, at least in part due to the angling/offsetting of the tubes, has a high deposition rate and retention rate of aerosolized particles that include the microbes on the photocatalytic coating catalyst, even at low airflow rate, e.g., less than 40 ACH, preferably less than 30 ACH, and most preferably between 5 and 20 ACH. By having such features, the surface area of the photocatalytic coating catalyst having the aerosolized particles that include the microbes is increased, so that the photocatalytic coating catalyst can further oxidize and/or decompose the microbes, e.g., due to the increased surface area, exposure to the light source is increased to further activate the photocatalytic coating catalyst and neutralize the virions.

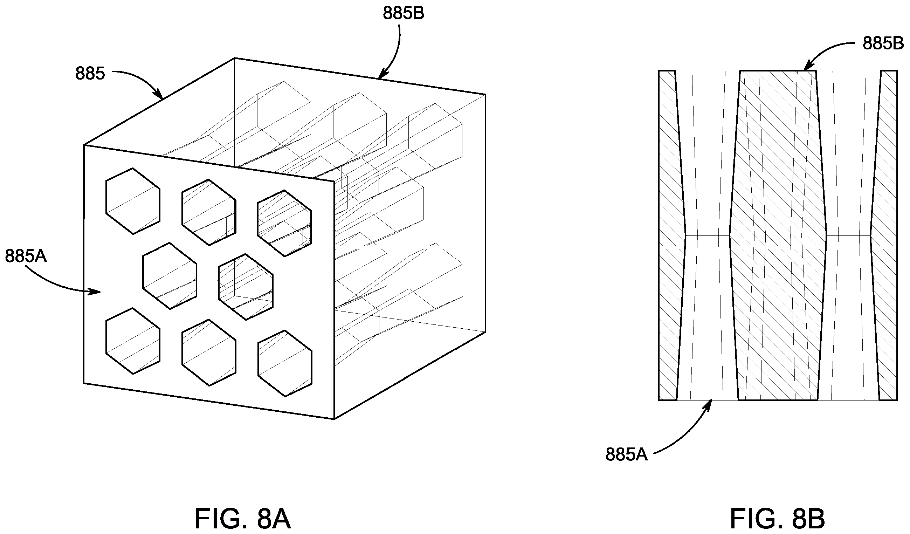

FIGS. 8A and 8B show a schematic representation of a substrate according to another embodiment. As seen in FIG. 8A, the substrate 885 has a honeycomb structure that includes a plurality of cells that define channels that extend along the flow path of the airflow. The plurality of cells defining the channels can include hexagonal tubes that are covered with the photocatalytic catalyst. The honeycomb structure can be a metal substrate, and can include metals such as aluminum, copper, steel, stainless, steel, alloys thereof, or combinations thereof or a ceramic or plastic substrate, or similar materials that are usable for photocatalytic oxidation substrates. The at least one photocatalytic coating can include a photocatalytic catalyst that can create hydroxyl radicals, H₂O₂, ions, super-oxides ions, or the like that can oxidize and/or decompose any VOCs and microbes. In some embodiments, the at least one photocatalytic coating is a graphene enhanced titanium oxide catalyst to generate local hydroxyl radicals and super-oxide ions on the surface of the catalyst that can oxidize and/or decompose VOCs and microbes when they come in contact with the substrate 885.

As seen in FIG. 8A, the plurality of cells has a structure in which the hexagonal tubes have an inlet end 885A and an outlet end 885B. As seen in FIG. 8B, the hexagonal tubes are tapered between the inlet end 885A and the outlet end 885B by converging near a center point of the substrate and then diverge towards the outlet end 885B so that the hexagonal tubes have a smaller cell diameter near the center point of the substrate than the cell diameter at the inlet end 885A and the outlet end 885B. In view of such structure, it is appreciated that the substrate structure defining the channels, at least in part due to the converging of the tubes near the center of the substrate which restricts airflow, has a high deposition rate and retention rate of aerosolized particles that include the microbes on the photocatalytic coating catalyst, even at low airflow rate, e.g., less than 40 ACH, preferably less than 30 ACH, and most preferably between 5 and 20 ACH. By having such features, the surface area of the photocatalytic coating catalyst having the aerosolized particles that include the microbes is increased, so that the photocatalytic coating catalyst can further oxidize and/or decompose the microbes, e.g., due to the increased surface area, exposure to the light source is increased to further activate the photocatalytic coating catalyst and neutralize the virions. It is appreciated that the convergence of the hexagonal tube can occur at the center of the substrate, but can also occur along other points or portions of the channel along the flow direction of the airflow to have similar effects.

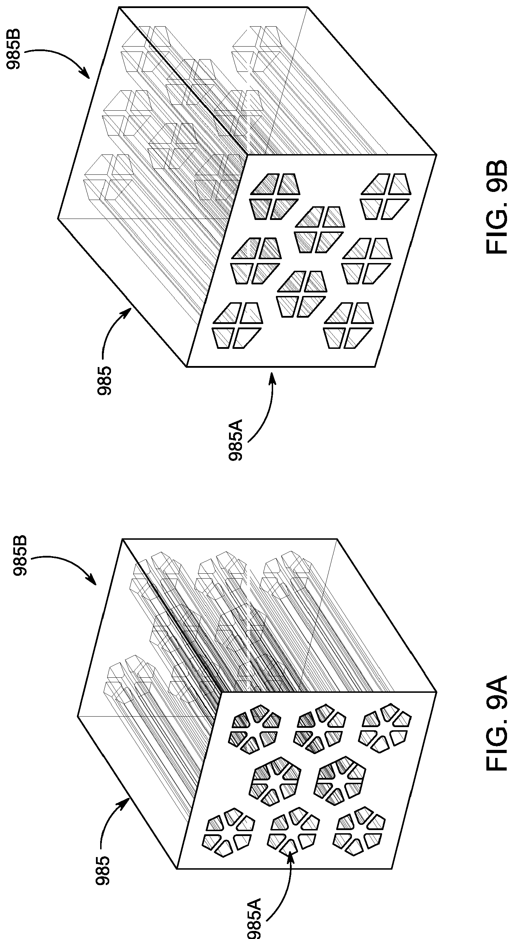

FIGS. 9A and 9B show a schematic representation of a substrate according to another embodiment. As seen in FIGS. 9A and 9B, the substrate 985 has a honeycomb structure that includes a plurality of cells that define channels that extend along the flow path of the airflow. The plurality of cells defining the channels can include hexagonal tubes that are covered with the photocatalytic catalyst. The honeycomb structure can be a metal substrate, and can include metals such as aluminum, copper, steel, stainless, steel, alloys thereof, or combinations thereof or a ceramic or plastic substrate, or similar materials that are usable for photocatalytic oxidation substrates. The at least one photocatalytic coating can include a photocatalytic catalyst that can create hydroxyl radicals, H₂O₂, ions, super-oxides ions, or the like that can oxidize and/or decompose any VOCs and microbes. In some embodiments, the at least one photocatalytic coating is a graphene enhanced titanium oxide catalyst to generate local hydroxyl radicals and super-oxide ions on the surface of the catalyst that can oxidize and/or decompose VOCs and microbes when they come in contact with the substrate 985.

As seen in FIGS. 9A and 9B, the plurality of cells has a structure in which the hexagonal tubes have an inlet end 985A and an outlet end 985B. Each of the cells of the plurality of cells include multiple units to form each of the cells, in which each of the multiple units define the channels that extend along the flow path of the airflow. For example, FIG. 9A shows that the cell has a hexagonal shape and six units are combined to form the cell. Similarly, FIG. 9B shows the cell having an octagonal shape, in which four units are combined to form the cell. In view of such structures, it is appreciated that the substrate structure, at least in part due to the multiple units forming each of the cells, which increases the surface area of contact of the surface of the catalyst, has a high deposition rate and retention rate of aerosolized particles that include the microbes on the photocatalytic coating catalyst, even at low airflow rate, e.g., less than 40 ACH, preferably less than 30 ACH, and most preferably between 5 and 20 ACH. By having such features, the surface area of the photocatalytic coating catalyst having the aerosolized particles that include the microbes is increased, so that the photocatalytic coating catalyst can further oxidize and/or decompose the microbes, e.g., due to the increased surface area, exposure to the light source is increased to further activate the photocatalytic coating catalyst and neutralize the virions.

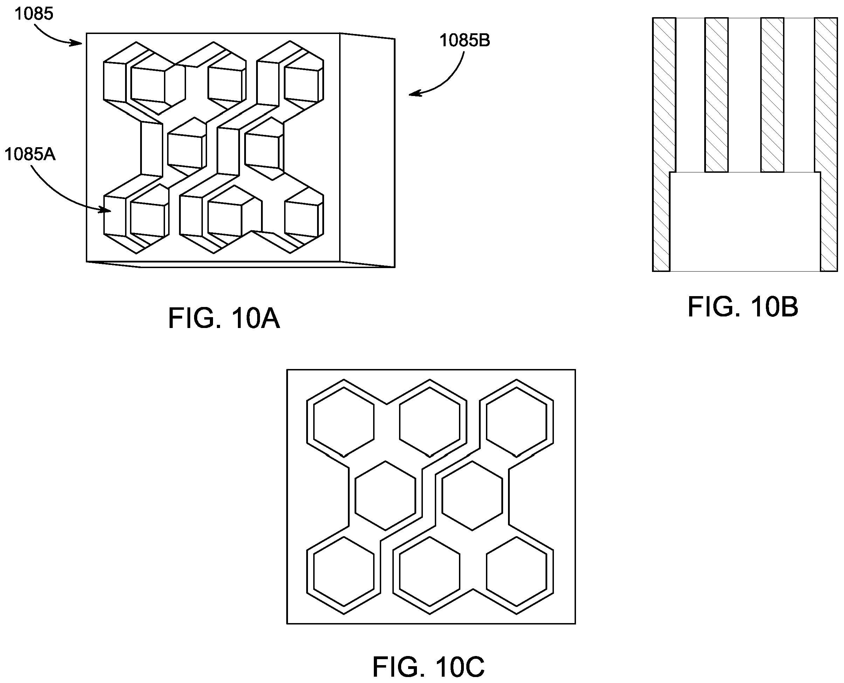

FIGS. 10A, 10B, 10C show a schematic representation of a substrate according to another embodiment. As seen in FIG. 10A, the substrate 1085 has a honeycomb structure that includes a plurality of cells that define channels that that extend along the flow path of the airflow. The plurality of cells defining the channels can include hexagonal tubes that are covered with the photocatalytic catalyst. The honeycomb structure can be a metal substrate, and can include metals such as aluminum, copper, steel, stainless, steel, alloys thereof, or combinations thereof or a ceramic or plastic substrate, or similar materials that are usable for photocatalytic oxidation substrates. The at least one photocatalytic coating can include a photocatalytic catalyst that can create hydroxyl radicals, H₂O₂, ions, super-oxides ions, or the like that can oxidize and/or decompose any VOCs and microbes. In some embodiments, the at least one photocatalytic coating is a graphene enhanced titanium oxide catalyst to generate local hydroxyl radicals and super-oxide ions on the surface of the catalyst that can oxidize and/or decompose VOCs and microbes when they come in contact with the substrate 1085.

As seen in FIG. 10A, the plurality of cells has a structure in which the hexagonal tubes have an inlet end 1085A and an outlet end 1085B. As seen in FIGS. 10B and 10C, the hexagonal tubes include a first plurality of cells and a second plurality of cells, in which the cells of both the first and second plurality of cells are stepped at a depth from a front face of the inlet end 1085A. In view of such structure, it is appreciated that the substrate structure, at least in part due to the stepped depth of the tubes at the inlet end, increases the surface area exposure to the light source. Thus, since the surface area of the catalyst having the aerosolized particles that include the microbes exposed to the light source is increased, the photocatalytic coating catalyst can further oxidize and/or decompose the microbes, e.g., due to the increased surface area, exposure to the light source is increased to further activate the photocatalytic coating catalyst and neutralize the virions. It is appreciated that the stepped depth of the cells can occur at the inlet end, as well as the outlet end, or combination thereof, which have similar effects and improvements.

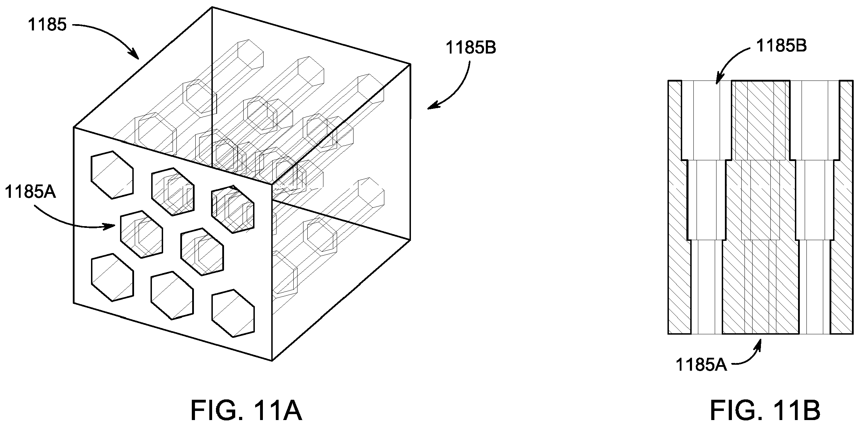

FIGS. 11A and 11B shows a schematic representation of a substrate according to another embodiment. The substrate 1185 has a honeycomb structure that includes a plurality of cells that defines channels that extend along the flow path of the airflow. The plurality of cells defining the channels can include hexagonal tubes that are covered with the photocatalytic catalyst. The honeycomb structure can be a metal substrate, and can include metals such as aluminum, copper, steel, stainless, steel, alloys thereof, or combinations thereof or a ceramic or plastic substrate, or similar materials that are usable for photocatalytic oxidation substrates. The at least one photocatalytic coating can include a photocatalytic catalyst that can create hydroxyl radicals, H₂O₂, ions, super-oxides ions, or the like that can oxidize and/or decompose any VOCs and microbes. In some embodiments, the at least one photocatalytic coating is a graphene enhanced titanium oxide catalyst to generate local hydroxyl radicals and super-oxide ions on the surface of the catalyst that can oxidize and/or decompose VOCs and microbes when they come in contact with the substrate 1185.

As seen in FIGS. 11A and 11B, the plurality of cells has a structure in which the hexagonal tubes have an inlet end 1185A and an outlet end 1185B. The inlet end 1185A of the hexagonal tubes has a cell diameter that is larger than the cell diameter at the outlet end 1185B. For example, the cells at the outlet end 1185B can have a cell diameter that is 1/4, 1/3, 1/2, 3/4, or the like the size of the cell diameter at the inlet end 585A. While similar to the design of the substrate shown in FIG. 5, the hexagonal tubes taper from the inlet end 585A to the outlet end 585B, in this embodiment, the tapering occurs in stepped gradations, in which each step has a smaller diameter than the previous step. While not intending to be limiting in scope, each stepped gradation can be 50%, 60%, 70%, 80%, or 90% the cell diameter of the previous portion and can have a length that is equal or different. In view of such structure, it is appreciated that the substrate structure, at least in part due to the narrowing/tapering of the tubes and the stepped portion which restrict airflow, has a high deposition rate and the retention rate of aerosolized particles that include the microbes on the photocatalytic coating catalyst, even at low airflow rate, e.g., less than 40 ACH, preferably less than 30 ACH, and most preferably between 5 and 20 ACH. By having such features, the surface area of the photocatalytic coating catalyst having the aerosolized particles that include the microbes is increased, so that the photocatalytic coating catalyst can further oxidize and/or decompose the microbes, e.g., due to the increased surface area, exposure to the light source is increased to further activate the photocatalytic coating catalyst and neutralize the virions.

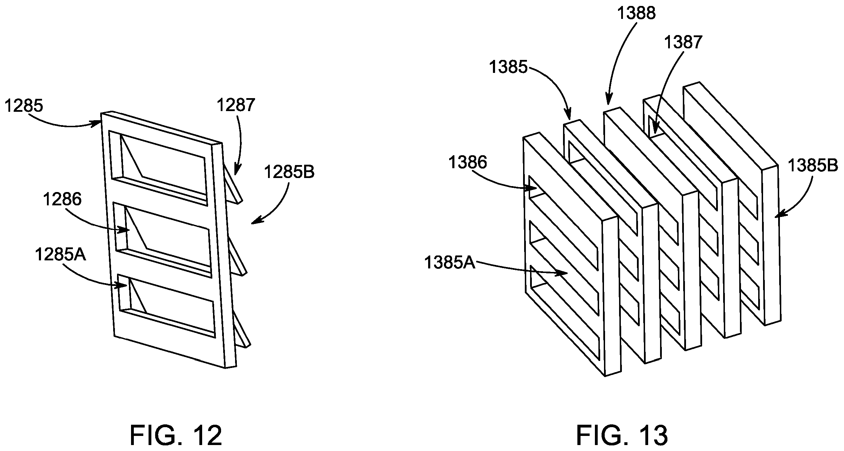

FIG. 12 shows a schematic representation of a substrate according to another embodiment. The substrate 1285 has a plate structure that includes a plurality of opening slots 1286 and deflectors 1287 that define channels that extend along the flow path of the airflow. At least one photocatalytic coating is on at least one surface of at least the deflectors, but can also be provided on the front face of the substrate at the inlet end. The plate structure can be a metal substrate, and can include metals such as aluminum, copper, steel, stainless, steel, alloys thereof, or combinations thereof or a ceramic or plastic substrate, or similar materials that are usable for photocatalytic oxidation substrates. The at least one photocatalytic coating can include a photocatalytic catalyst that can create hydroxyl radicals, H₂O₂, ions, super-oxides ions, or the like that can oxidize and/or decompose any VOCs and microbes. In some embodiments, the at least one photocatalytic coating is a graphene enhanced titanium oxide catalyst to generate local hydroxyl radicals and super-oxide ions on the surface of the catalyst that can oxidize and/or decompose VOCs and microbes when they come in contact with the substrate 1285.

As seen in FIG. 12, the plate substrate 1285 has an inlet end 1285A and an outlet end 1285B. The inlet end 1285A of the plate substrate 1285 includes the slot openings 1286, in which each deflector 1287 is provided at the respective slot openings 1286 at the outlet end 1285B, which together define the channels. It is appreciated that as airflow flows through the slot openings 1286, the airflow is deflected by the deflector 1287 having the at least one photocatalytic coating for capturing microbes in the airflow. In view of such structure, it is appreciated that the substrate structure, at least in part due to the deflection of the airflow by the deflector 1287, has a high deposition rate and the retention rate of aerosolized particles that include the microbes on the photocatalytic coating catalyst, even at low airflow rate, e.g., less than 40 ACH, preferably less than 30 ACH, and most preferably between 5 and 20 ACH. By having such features, the surface area of the photocatalytic coating catalyst having the aerosolized particles that include the microbes is increased, so that the photocatalytic coating catalyst can further oxidize and/or decompose the microbes, e.g., due to the increased surface area, exposure to the light source is increased to further activate the photocatalytic coating catalyst and neutralize the virions.

It is appreciated that FIG. 12 shows that the deflectors 1287 are plate-shaped deflectors that are angled to deflect the airflow and increase the contact with the surface area of the catalyst. In an embodiment, the plate-shaped deflectors are adjustable in that the plate-shaped deflectors 1287 can be controlled to adjust the angle or degree of opening of the slot openings 1286, e.g., using the controller 255. For example, the plate-shaped deflectors can be adjusted using solenoids or geared motors to control the angle of the plate-shaped deflectors based in the airflow rate, e.g., at low airflow rates, the plate-shaped deflectors can be angled to decrease the size of the opening of the slot openings, e.g., between 10 and 30 degrees with respect to the plate substrate, and as airflow rates increase, the plate-shaped deflectors can be angled to increase the size of the opening of the slot openings, e.g., between 60 degrees and 80 degrees with respect to the plate substrate.

FIG. 13 shows a schematic representation of a substrate according to another embodiment. The embodiment of FIG. 13 is similar to the embodiment of FIG. 12, but includes a plurality of the plates. Specifically, the substrate 1385 includes a plurality of plate structures that includes a plurality of opening slots 1386 and deflectors 1387 that define channels that extend along the flow path of the airflow. At least one photocatalytic coating is on at least one surface of at least the deflectors, but can also be provided on the front face of the substrate at the inlet end. The plate structure can be a metal substrate, and can include metals such as aluminum, copper, steel, stainless, steel, alloys thereof, or combinations thereof or a ceramic or plastic substrate, or similar materials that are usable for photocatalytic oxidation substrates. The at least one photocatalytic coating can include a photocatalytic catalyst that can create hydroxyl radicals, H₂O₂, ions, super-oxides ions, or the like that can oxidize and/or decompose any VOCs and microbes. In some embodiments, the at least one photocatalytic coating is a graphene enhanced titanium oxide catalyst to generate local hydroxyl radicals and super-oxide ions on the surface of the catalyst that can oxidize and/or decompose VOCs and microbes when they come in contact with the substrate 1385.

As seen in FIG. 13, the substrate 1285 has an inlet end 1285A and an outlet end 1285B. Each of the plates 1388 include the slot openings 1386, in which each deflector 1387 is provided at the respective slot openings 1386. The plate 1388 can be arranged in series so that the airflow flows through the slot openings 1386 of the first plate 1388 in series and then subsequently through the remaining plates, in which the airflow is deflected by the deflectors 1387 having the at least one photocatalytic coating for capturing microbes in the airflow. In view of such structure, it is appreciated that the substrate structure, at least in part due to the deflection of the airflow by the deflectors 1387, has a high deposition rate and the retention rate of aerosolized particles that include the microbes on the photocatalytic coating catalyst, even at low airflow rate, e.g., less than 40 ACH, preferably less than 30 ACH, and most preferably between 5 and 20 ACH. By having such features, the surface area of the photocatalytic coating catalyst having the aerosolized particles that include the microbes is increased, so that the photocatalytic coating catalyst can further oxidize and/or decompose the microbes, e.g., due to the increased surface area, exposure to the light source is increased to further activate the photocatalytic coating catalyst and neutralize the virions. It is appreciated that while the substrate 1385 is discussed with respect to the plates 1388 being in series, different arrangements of the plates 1388 are contemplated, for example, the plates 1388 can be staggered in series to provide less flow restriction between plates 1388.

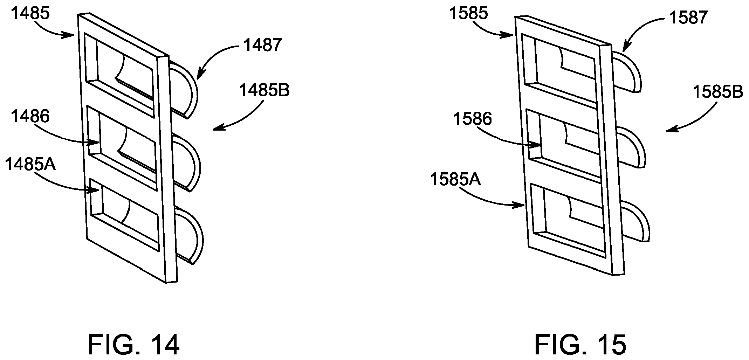

FIG. 14 shows a schematic representation of a substrate according to another embodiment. The substrate 1485 has a plate structure that includes a plurality of opening slots 1486 and deflectors 1487 that define channels that extend along the flow path of the airflow. At least one photocatalytic coating is on at least one surface of at least the deflectors, but can also be provided on the front face of the substrate at the inlet end. The plate structure can be a metal substrate, and can include metals such as aluminum, copper, steel, stainless, steel, alloys thereof, or combinations thereof or a ceramic or plastic substrate, or similar materials that are usable for photocatalytic oxidation substrates. The at least one photocatalytic coating can include a photocatalytic catalyst that can create hydroxyl radicals, H₂O₂, ions, super-oxides ions, or the like that can oxidize and/or decompose any VOCs and microbes. In some embodiments, the at least one photocatalytic coating is a graphene enhanced titanium oxide catalyst to generate local hydroxyl radicals and super-oxide ions on the surface of the catalyst that can oxidize and/or decompose VOCs and microbes when they come in contact with the substrate 1485.

As seen in FIG. 14, the plate substrate 1485 has an inlet end 1485A and an outlet end 1485B. The inlet end 1485A of the plate substrate 1485 includes the slot openings 1486, in which each deflector 1487 is provided at the respective slot openings 1486 at the outlet end 1485B, which together define the channels. The deflectors 1487 have a semi-circular design, e.g., a scoop-design, that is designed to deflect the airflow at least partially on the backside of the corresponding deflector 1487 as the airflow flows through the slot openings 1486. Thus, the deflector 1487 includes the at least one photocatalytic coating on a frontside of the deflector 1487, as well as on the backside, so that the at least one photocatalytic coating can capture microbes in the airflow. In view of such structure, it is appreciated that the substrate structure, at least in part due to the deflection of the airflow by the deflector 1287 and the additional surfaces having the at least one photocatalytic coating, has a high deposition rate and the retention rate of aerosolized particles that include the microbes on the photocatalytic coating catalyst, even at low airflow rate, e.g., less than 40 ACH, preferably less than 30 ACH, and most preferably between 5 and 20 ACH. By having such features, the surface area of the photocatalytic coating catalyst having the aerosolized particles that include the microbes is increased, so that the photocatalytic coating catalyst can further oxidize and/or decompose the microbes, e.g., due to the increased surface area, exposure to the light source is increased to further activate the photocatalytic coating catalyst and neutralize the virions. It is appreciated that this design also allows an illumination arrangement in which the surface area that is exposed to the light source is increased, which increases the yield of reactive oxygen species for neutralizing the virions.

FIG. 15 shows a schematic representation of a substrate according to another embodiment. The substrate 1585 has a plate structure that includes a plurality of opening slots 1586 and deflectors 1587 that define channels that extend along the flow path of the airflow. At least one photocatalytic coating is on at least one surface of at least the deflectors, but can also be provided on the front face of the substrate at the inlet end. The plate structure can be a metal substrate, and can include metals such as aluminum, copper, steel, stainless, steel, alloys thereof, or combinations thereof or a ceramic or plastic substrate, or similar materials that are usable for photocatalytic oxidation substrates. The at least one photocatalytic coating can include a photocatalytic catalyst that can create hydroxyl radicals, H₂O₂, ions, super-oxides ions, or the like that can oxidize and/or decompose any VOCs and microbes. In some embodiments, the at least one photocatalytic coating is a graphene enhanced titanium oxide catalyst to generate local hydroxyl radicals and super-oxide ions on the surface of the catalyst that can oxidize and/or decompose VOCs and microbes when they come in contact with the substrate 1585.

As seen in FIG. 15, the plate substrate 1585 has an inlet end 1585A and an outlet end 1585B. The inlet end 1585A of the plate substrate 1585 includes the slot openings 1586, in which each deflector 1587 is provided at the respective slot openings 1586 at the outlet end 1585B, which together define the channels. The deflectors 1587 have a quarter-circular design, e.g., a half scoop-design, that is designed to deflect the airflow at least partially on the backside of the corresponding deflector 1587 as the airflow flows through the slot openings 1586. Thus, the deflector 1587 includes the at least one photocatalytic coating on a frontside of the deflector 1587, as well as on the backside, so that the at least one photocatalytic coating capture microbes in the airflow. In view of such structure, it is appreciated that the substrate structure, at least in part due to the deflection of the airflow by the deflector 1587 and the additional surfaces having the at least one photocatalytic coating, has a high deposition rate and retention rate of aerosolized particles that include the microbes on the photocatalytic coating catalyst, even at low airflow rate, e.g., less than 40 ACH, preferably less than 30 ACH, and most preferably between 5 and 20 ACH. By having such features, the surface area of the photocatalytic coating catalyst having the aerosolized particles that include the microbes is increased, so that the photocatalytic coating catalyst can further oxidize and/or decompose the microbes, e.g., due to the increased surface area, exposure to the light source is increased to further activate the photocatalytic coating catalyst and neutralize the virions. It is appreciated that this design also allows an illumination arrangement in which the surface area that is exposed to the light source is increased, which increases the yield of reactive oxygen species for neutralizing the virions.

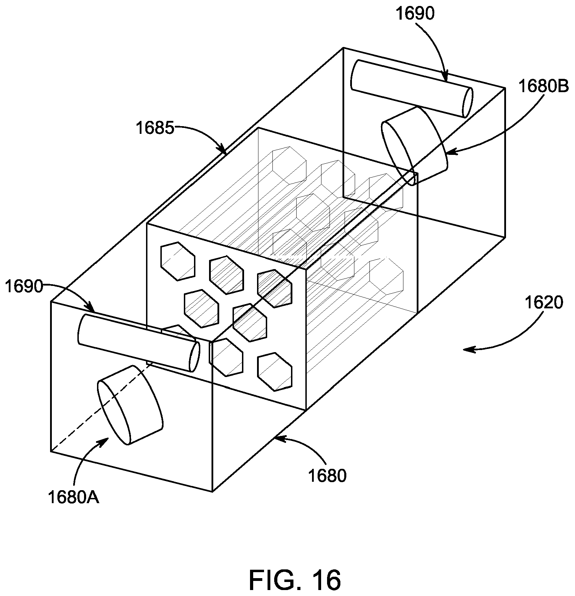

FIG. 16 shows a schematic representation of an embodiment of an air sanitizer unit 1620 which can be used in any of the systems as discussed above with respect to air sanitizer units 220, 320, 420. The air sanitizer unit 1620 includes a housing 1680, a substrate 1685 provided in an interior space of the housing 1680, and at least two light sources 1690 disposed within the interior space of the housing 1680. The housing 1680 can include an inlet 1680A for receiving the airflow from the climate controlled space and/or a filter and an outlet 1680B to discharge the purified and/or sanitized airflow to be conditioned and/or back into the climate controlled space. It is appreciated that the inlet 1680A and/or the outlet 1680B can be an aperture or hole provided on a front face of the air sanitizer unit 1620 that allows the airflow to enter and/or exit, or can be the entirety of the front face and/or back face of the air sanitizer unit 1620.

The substrate 1685 includes at least one photocatalytic coating on at least one surface that extends along a flow path of the airflow through the air sanitizer unit 1620. For example, without intending to be limiting in scope, the substrate 1685 can have a honeycomb structure, in which the honeycomb structure includes a plurality of cells that define channels that extend along a flow path of the airflow through the air sanitizer unit 1620 or a plate structure, in which the plate structure includes deflectors that define the channels that extend along a flow path of the airflow through the air sanitizer unit 1620.

The at least two light sources 1690 are disposed within the interior space of the housing in a way such that light produced by the light source activates the at least one photocatalytic coating to purify and/or sanitize the airflow, e.g., shines in the same direction of airflow. The light sources 1690 can use an ultraviolet (UV) light to excite and activate the catalyst (e.g., a graphene enhanced titanium oxide catalyst) to begin the chemical reaction. The use of graphene can increase the amount of surface area that the titanium oxide is applied to and thus create more surface area for UV light to shine, thereby increasing the amount of hydroxyl radicals and/or super-oxide ions generated. In some embodiments, the UV light can be generated using one or more light emitting diodes (LEDs), one or more mercury lamps, etc. In some embodiments, the UV light can generate ~390 nm light (e.g., when using one or more LEDs, the UV light generated can spike at around 395 nm). By illuminating the substrate at the inlet end and the outlet end, an increase in the surface area of light exposure of the substrate is provided, which increases the yield of reactive oxygen species for neutralizing the virions. It is appreciated that while the light sources 1690 are discussed with respect to be provided at the inlet end and the outlet end of the substrate, it is understood that the light sources can include a plurality of discrete light sources positioned in the housing to increase the surface area of light exposure, as necessary. For example, in the case of a plate substrate, the light sources can be provided above and/or below the deflectors to maximize the surface area of light exposure and/or a light source can be provided for each cell for a honeycomb substrate or each deflector for a plate substrate.

It is appreciated that while the different designs of the substrates have been discussed above as separate substrates or in different air sanitizer units, it is understood that the discussion is not intended to limit the scope of the disclosure, but instead, the various features of the substrates and/or air sanitizer units can be combined or modified to further increase the surface area for exposure to the light source to further increase the antimicrobial performance of the air sanitizer unit, as necessary.

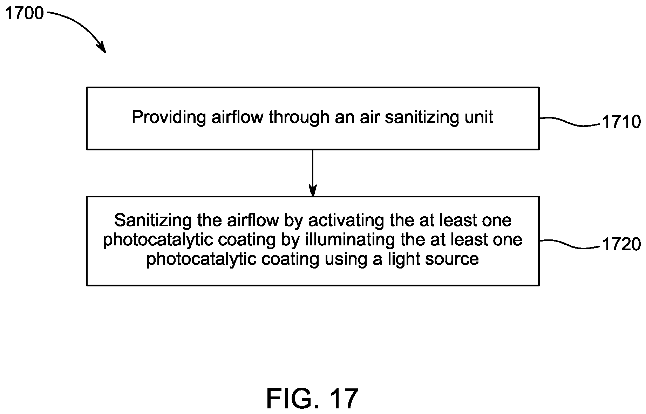

Operation of the air sanitizer unit is described below with respect to FIG. 17.

FIG. 17 illustrates a flowchart for a method 1700 for sanitizing air in any of the climate control systems, as discussed above in FIGS. 1A-3, according to an embodiment.

The method 1700 begins at 1710 whereby airflow is provided through an air sanitizing unit. The air sanitizing unit includes a housing, a substrate provided in an interior space of the housing, the substrate including at least one photocatalytic coating on at least one surface that extends along a flow path of airflow through the air sanitizing unit, and a light source disposed within the interior space of the housing. The substrate includes channels for allowing airflow there through, in which a substrate structure defining the channels include the photocatalytic coating and the substrate structure defining the channels is configured such that microbes in the airflow are captured on the least one photocatalytic coating at low airflow rates. For example, the substrate structure can include a honeycomb structure that includes a plurality of cells that define channels, in which the plurality of cells that define the channels include hexagonal tubes that are covered with the photocatalytic catalyst, or plate structure that include opening slots and deflectors, which define the channels, in which the at least the deflectors are covered with the photocatalytic catalyst. In view of such structural designs, microbes in the airflow are capture more efficiently, e.g., high deposition rate and retention rate, even at low airflow rates, e.g., less than 40 ACH, preferably less than 30 ACH, and most preferably between 5 and 20 ACH. The method 1700 proceeds to 1720,

At 1720, the airflow is sanitized by activating the at least one photocatalytic coating by illuminating the at least one photocatalytic coating using the light source. Since the surface area of the catalyst having the aerosolized particles that include the microbes is increased, the catalyst can further oxidize and/or decompose the microbes, e.g., due to the increased surface area, exposure to the light source is increased to further activate the photocatalytic coating catalyst and neutralize the virions.

It is appreciated that a controller (e.g., the climate controller 170, 255 shown in FIGS. 1C and 2) can be used to control the climate control system and also control operation of the air sanitizer unit. In particular, the controller can control operation of the climate control system by controlling operation of the air movement fans/blowers, the compressor, the one or more condenser fans, valves, etc. That is, the controller can control whether the air movement fans/blowers, the compressor, the one or more condenser fans, one or more valves, etc. are ON or OFF. The controller can also control a speed of the air movement fans/blowers, the compressor, the one or more condenser fans, etc. Also, the controller can control the size of the opening of the one or more valves. The controller is configured to receive climate control parameter data from the one or more climate control sensors. It will be appreciated that the controller can use the climate control parameter data to control operation of the climate control system and the air sanitization unit.

The controller can also be in electrical communication with the air sanitizer unit. Accordingly, the controller can turn the air sanitizer unit ON or OFF as required and control the amount of power directed to the air sanitizer unit. In some embodiments, the controller can selectively turn ON and OFF or limit the power provided to individual LED panels of the air sanitizer unit while the air sanitizer unit is in operation. For example, in some embodiments, the controller can selectively turn OFF or limit power directed to a certain number of the LED panels while the air sanitizer unit is in operation in order to reduce the purification capacity of the air sanitizer unit. The controller can selectively turn OFF or reduce power to a certain number of the LED panels while the air sanitizer unit is operating in order to reduce energy consumption of the air sanitizer unit during, for example, periods when the mass-transit vehicle may be lightly loaded, the risk of contamination in the climate controlled space is low, etc. Also, the controller can rotate which of the multiple LED panels are ON and OFF or have reduced power to achieve uniform aging of the air sanitizer unit. In some embodiments, the controller is configured to receive operational parameter data from the one or more operational sensors.

In an embodiment, the controller can receive data on the airflow rate flowing through the climate control system. Thus, if the airflow rate is at a low airflow rate, e.g., between 5 ACH and 20 ACH, the controller can control operation of the substrate structure, if available. For example, when the substrate structure includes the plate structure having the opening slots and deflectors, the controller can control the angle of the deflectors based on the airflow rate. It is appreciated that the control of the angle of the deflectors can also be based on other operational parameter data, for example, based on low occupancy or low sensed VOCs and/or microbe in the airflow.

The discussion herein relates to flow rates expressed by reference to ACH (air changes per hour) and corresponding configuration of an air sanitizer unit (e.g. a substrate structure) to capture microbes at low flow rates. Any flow rate expressed herein by reference to ACH may be converted to units of m³/s, for example for an occupied space (e.g. a controlled space for sanitization) with a volume of 700m³. For example, a low airflow rate of less than 40 ACH, preferably less than 30 ACH, and most preferably between 5 and 20 ACH corresponds to volumetric airflow rates of less than 7.78m³/s (40 ACH), preferably less than 5.83m³/s (30 ACH), and most preferably between 0.97m³/s and 3.89m³/s for an occupied space with a volume of 700m³. Further, a sanitizer unit (e.g. a substrate structure of a sanitizer unit) as described herein may be configured to capture microbes in the airflow on the at least one photocatalytic coating and otherwise function as described herein at low volumetric airflow rates corresponding to the ranges defined above in units of m³/s (i.e. less than 7.78m³/s, preferably less than 5.83m³/s, and most preferably between 0.97m³/s and 3.89m³/s).

Further, the disclosure envisages installation of an air sanitizer unit as described herein, for example as part of a climate control system, with (e.g. in fluid communication with) an occupied space (e.g. a controlled space for sanitization), such that at the low airflow rates expressed elsewhere herein (e.g. a low airflow rate of less than 40 ACH, preferably less than 30 ACH, and most preferably between 5 and 20 ACH), the substrate structure defining the channels captures microbes in the airflow on the at least one photocatalytic coating.

### RESULTS

Several of the different designs of the substrates were tested to test the amount of trapped particles on the substrate. Specifically, the substrate having the honeycomb design without any modifications (FIG. 4) was compared with the plate substrate designs including the plate-shaped deflector design (FIG. 12), the scoop deflector design (FIG. 14), and the half-scoop deflector design (FIG. 15).

As discussed above, it is understood that the size of a VOC gas molecule, for example, a formaldehyde molecule, is about 0.25 nm, while the size of a pathogen, for example, the MS2 virus (which is a small virus), is 27 nm. In other words, the size of the microbe is 100 times larger than the VOC gas molecule, and has a volume ratio of 1,000,000: 1. Further, when one considers that virions are rarely found individually and that they are typically found in aerosolized particles of size 1 µm or larger, the volume of aerosolized particles carrying microbes may be 64 billion (or more) times larger than that of VOC molecules.

Accordingly, the different substrate designs were tested with different sized particle diameters to test the trapping efficiency of the substrate having the at least one photocatalytic coating, as seen in the following table:

**TABLE 1**

| | Min Dia | Mean Dia | Max Dia |
|---|---|---|---|
| Case 1 | 30 nm | 0.5 micron | 1 micron |
| Case 2 | 1 micron | 5 micron | 10 micron |
| Case 3 | 10 micron | 30 micron | 50 micron |
| Case 4 | 50 micron | 75 micron | 100 micron |

The different substrate designs were tested at an inlet flowrate of 500 fpm , an outlet pressure of 0 PA, and water mass flowrate of 1 e-7 kg/s. As seen in the following table, the plate substrate designs resulted in a greater percentage of trapped mass as the particle diameters increased. Thus, since a greater percent of the aerosolized particles having the microbes are trapped on the surface of the photocatalytic coating, the antimicrobial performance of such plate substrates is greater than the unmodified honeycomb substrate design.

**TABLE 2**

| | Baseline | Baffle | Scoop | Half Scoop |
|---|---|---|---|---|
| Case 1 | 2% | 4% | 3% | 3% |
| Case 2 | 5% | 17% | 16% | 7% |
| Case 3 | 49% | 100% | 98% | 77% |
| Case 4 | 60% | 100% | 99% | 84% |

The terminology used in this Specification is intended to describe particular embodiments and is not intended to be limiting. The terms "a," "an," and "the" include the plural forms as well, unless clearly indicated otherwise. The terms "comprises" and/or "comprising," when used in this Specification, specify the presence of the stated features, integers, steps, operations, elements, and/or components, but do not preclude the presence or addition of one or more other features, integers, steps, operations, elements, and/or components.

With regard to the preceding description, it is to be understood that changes can be made in detail, especially in matters of the construction materials employed and the shape, size, and arrangement of parts without departing from the scope of the invention as defined by the appended claims. For example, while the air sanitizer unit has been discussed above with respect to the air sanitizer unit being used with a climate control system for a transport unit or HVAC applications, it is understood that the air sanitizer unit can be a standalone unit. This specification and the embodiments described are exemplary only, with the true scope of the invention being indicated by the claims that follow.

## Claims

1. An air sanitizer unit (420) comprising:
a housing (480);
a substrate (485) provided in an interior space of the housing, the substrate comprising at least one photocatalytic coating on at least one surface that extends along a flow path of airflow through the air sanitizing unit;
a light source (490) disposed within the interior space of the housing in a way such that light produced by the light source activates the at least one photocatalytic coating to purify the airflow,
wherein the substrate comprises a honeycomb cell structure that includes a first plurality of cells defining channels that extend along the flow path of the airflow for allowing airflow there through, wherein the channels include the photocatalytic coating and the channels are configured to capture microbes in the airflow on the at least one photocatalytic coating, and
wherein the first plurality of cells each has an inlet end and an outlet end,
**characterized in that**:
the inlet end has a cell diameter larger than a cell diameter of the outlet end to restrict flow through the air sanitizing unit.

2. The air sanitizer unit of claim 1, wherein the channels are configured such that microbes in the airflow are captured on the at least one photocatalytic coating at low airflow rates;
wherein the low airflow rates are airflow rates less than 20 air changes per hour.

3. The air sanitizer unit of claim 1 or 2, wherein the at least one photocatalytic coating is a graphene-enhanced photocatalytic oxidation coating comprising graphene enhanced titanium oxide catalyst.

4. The air sanitizer unit of any of claims 1 to 3, wherein the light source comprises a first light source (1690) and a second light source (1690), wherein the first light source is provided in the interior space of the housing to produce light upstream of the substrate structure and the second light source is provided in the interior space of the housing to produce light downstream of the substrate structure.

5. The air sanitizer unit of claim 1,
wherein each of the first plurality of cells comprises multiple units that form each of the plurality of cells, wherein each of the multiple units define channels having the surfaces that extend along the flow path of the airflow.

6. The air sanitizer unit of claim 1, further comprising a second plurality of cells having an inlet end having the cell diameter smaller than the cell diameter of an outlet end.

7. The air sanitizer unit of claim 1, wherein at least a portion of each of the cells along the flow path of the air flow has a diameter that is smaller than a diameter of another portion of the cell along the channel.

8. The air sanitizer unit of claim 1, wherein the outlet end is offset at an angle from the inlet end.

9. A climate control system comprising:
a heating and/or refrigeration system; and
an air sanitization system,
wherein the air sanitization system comprises an air sanitizer in accordance with any of claims 1-8.

10. A method for sanitizing air comprising:
providing airflow through an air sanitizing unit in accordance with any of claims 1-8 and sanitizing the airflow by activating the at least one photocatalytic coating by illuminating the at least one photocatalytic coating using the light source.

## Patentansprüche

1. Luftentkeimungseinheit (420), umfassend:
ein Gehäuse (480);
ein Substrat (485), das in einem Innenraum des Gehäuses bereitgestellt wird, wobei das Substrat mindestens eine photokatalytische Beschichtung auf mindestens einer Oberfläche umfasst, die sich entlang eines Strömungswegs des Luftstroms durch die Luftentkeimungseinheit erstreckt;
eine Lichtquelle (490), die im Innenraum des Gehäuses so angeordnet ist, dass das von der Lichtquelle produzierte Licht die mindestens eine photokatalytische Beschichtung aktiviert, um den Luftstrom zu reinigen;
wobei das Substrat eine Wabenzellstruktur umfasst, die eine erste Vielzahl von Zellen einschließt, welche Kanäle definieren, die sich entlang des Strömungswegs des Luftstroms erstrecken, um einen Luftstrom dort hindurch zu ermöglichen, wobei die Kanäle die photokatalytische Beschichtung einschließen und die Kanäle so ausgestaltet sind, dass sie Mikroben in dem Luftstrom auf der mindestens eine photokatalytische Beschichtung einfangen, und
wobei die erste Vielzahl von Zellen jeweils ein Einlass- und ein Auslassende aufweist,
**dadurch gekennzeichnet, dass**:
das Einlassende einen Zelldurchmesser aufweist, der größer als ein Zelldurchmesser des Auslassendes ist, um Strömung durch die Luftentkeimungseinheit zu beschränken.

2. Luftentkeimungseinheit nach Anspruch 1, wobei die Kanäle so ausgestaltet sind, dass Mikroben in dem Luftstrom auf der mindestens einen photokatalytischen Beschichtung bei niedrigen Luftstromraten eingefangen werden;
wobei die niedrigen Luftstromraten Luftstromraten von weniger als 20 Luftwechseln pro Stunde sind.

3. Luftentkeimungseinheit nach Anspruch 1 oder 2, wobei die mindestens eine photokatalytische Beschichtung eine graphenverstärkte photokatalytische Oxidationsbeschichtung ist, die einen graphenverstärkten Titanoxidkatalysator umfasst.

4. Luftentkeimungseinheit nach einem der Ansprüche 1 bis 3, wobei die Lichtquelle eine erste Lichtquelle (1690) und eine zweite Lichtquelle (1690) umfasst, wobei die erste Lichtquelle in dem Innenraum des Gehäuses bereitgestellt wird, um Licht stromaufwärts der Substratstruktur zu produzieren, und die zweite Lichtquelle in dem Innenraum des Gehäuses bereitgestellt wird, um Licht stromabwärts der Substratstruktur zu produzieren.

5. Luftentkeimungseinheit nach Anspruch 1,
wobei jede der ersten Vielzahl von Zellen mehrere Einheiten umfasst, die jede der Vielzahl von Zellen bilden, wobei jede der mehreren Einheiten Kanäle mit den Oberflächen definiert, die sich entlang des Strömungswegs des Luftstroms erstrecken.

6. Luftentkeimungseinheit nach Anspruch 1, ferner umfassend eine zweite Vielzahl von Zellen mit einem Einlassende, dessen Zelldurchmesser kleiner als der Zelldurchmesser eines Auslassendes ist

7. Luftentkeimungseinheit nach Anspruch 1, wobei mindestens ein Teil von jeder der Zellen entlang des Strömungswegs des Luftstroms einen Durchmesser aufweist, der kleiner als ein Durchmesser eines anderen Teils der Zelle entlang des Kanals ist.

8. Luftentkeimungseinheit nach Anspruch 1, wobei das Auslassende in einem Winkel zu dem Einlassende versetzt ist.

9. Klimatisierungssystem, umfassend:
ein Heiz- und/oder Kühlsystem; und
ein Luftentkeimungssystem,
wobei das Luftentkeimungssystem einen Luftentkeimer nach einem der Ansprüche 1-8 umfasst.

10. Verfahren zur Luftentkeimung, umfassend:
Bereitstellung von Luftstrom durch eine Luftentkeimungseinheit nach einem der Ansprüche 1-8 und Entkeimen des Luftstroms durch Aktivieren der mindestens einen photokatalytischen Beschichtung durch Beleuchten der mindestens einer photokatalytischen Beschichtung unter Verwendung der Lichtquelle.

## Revendications

1. Unité de désinfection d'air (420), comprenant :
un logement (480) ;
un substrat (485) prévu dans un espace intérieur du logement, le substrat comprenant au moins un revêtement photocatalytique sur au moins une surface qui s'étend le long d'un chemin d'écoulement d'un écoulement d'air à travers l'unité de désinfection d'air ;
une source de lumière (490) disposée à l'intérieur de l'espace intérieur du logement de manière telle que de la lumière produite par la source de lumière active l'au moins un revêtement photocatalytique pour purifier l'écoulement d'air,
dans laquelle le substrat comprend une structure de cellules en nid d'abeille qui inclut une première pluralité de cellules définissant des canaux qui s'étendent le long du chemin d'écoulement de l'écoulement d'air pour permettre l'écoulement d'air à travers ceux-ci, dans laquelle les canaux incluent le revêtement photocatalytique et les canaux sont configurés pour capturer des microbes dans l'écoulement d'air sur l'au moins un revêtement photocatalytique, et
dans laquelle la première pluralité de cellules ont chacune une extrémité d'entrée et une extrémité de sortie,
**caractérisée en ce que** :
l'extrémité d'entrée a un diamètre de cellule plus grand qu'un diamètre de cellule de l'extrémité de sortie pour limiter l'écoulement à travers l'unité de désinfection d'air.

2. Unité de désinfection d'air de la revendication 1, dans laquelle les canaux sont configurés de manière telle que des microbes dans l'écoulement d'air sont capturés sur l'au moins un revêtement photocatalytique à de bas débits d'écoulement d'air ;
dans laquelle les bas débits d'écoulement d'air sont des débits d'écoulement d'air inférieurs à 20 changements d'air par heure.

3. Unité de désinfection d'air de la revendication 1 ou 2, dans laquelle l'au moins un revêtement photocatalytique est un revêtement à oxydation photocatalytique amélioré par graphène comprenant un catalyseur en oxyde de titane amélioré par graphène.

4. Unité de désinfection d'air de quelconques des revendications 1 à 3, dans laquelle la source de lumière comprend une première source de lumière (1690) et une seconde source de lumière (1690), dans laquelle la première source de lumière est prévue dans l'espace intérieur du logement pour produire de la lumière en amont de la structure de substrat et la seconde source de lumière est prévue dans l'espace intérieur du logement pour produire de la lumière en aval de la structure de substrat.

5. Unité de désinfection d'air de la revendication 1, dans laquelle chacune de la première pluralité de cellules comprend de multiples unités qui forment chacune de la pluralité de cellules, dans laquelle chacune des multiples unités définit des canaux ayant les surfaces qui s'étendent le long du chemin d'écoulement de l'écoulement d'air.

6. Unité de désinfection d'air de la revendication 1, comprenant en outre une seconde pluralité de cellules ayant une extrémité d'entrée ayant le diamètre de cellule plus petit que le diamètre de cellule d'une extrémité de sortie.

7. Unité de désinfection d'air de la revendication 1, dans laquelle au moins une partie de chacune des cellules le long du chemin d'écoulement de l'écoulement d'air a un diamètre qui est plus petit qu'un diamètre d'une autre partie de la cellule le long du canal.

8. Unité de désinfection d'air de la revendication 1, dans laquelle l'extrémité de sortie est décalée à un angle par rapport à l'extrémité d'entrée.

9. Système de régulation de conditions ambiantes, comprenant :
un système de chauffage et/ou de réfrigération ; et
un système de désinfection d'air,
dans lequel le système de désinfection d'air comprend un désinfecteur d'air conformément à de quelconques des revendications 1 à 8.

10. Procédé de désinfection d'air, comprenant :
la fourniture d'un écoulement d'air à travers une unité de désinfection d'air conformément à de quelconques des revendications 1 à 8, et la désinfection de l'écoulement d'air en activant l'au moins un revêtement photocatalytique en illuminant l'au moins un revêtement photocatalytique en utilisant la source de lumière.
